# EUROPEAN PATENT APPLICATION

(11) **EP 4 507 333 A1**
(43) Date of publication of application: **12.02.2025**
(21) Application number: 22967566.5
(22) Date of filing: 07.12.2022
(51) Int. Cl.: H04R 29/00, A41H 1/02

(54) **WEARABLE DEVICE**

(71) Applicant: Shenzhen Shokz Co., Ltd., Shenzhen, Guangdong 518108 (CN)
(72) Inventor: ZHOU, Xin, Shenzhen, Guangdong 518108 (CN)
(74) Representative: Wang, Bo
(86) International application number: PCT/CN2022/137284
(87) International publication number: WO 2024/119406

(57) **Abstract**

Provided is a wearable device comprising pants and a sensor assembly fixed on the pants. The sensor assembly includes at least one circumferential strain sensor. The at least one circumferential strain sensor extends along a circumferential direction of a pant leg of the pants and is configured to measure a circumferential dimension of a leg of a user.

## Description

### TECHNICAL FIELD

The present disclosure relates to the field of wearable devices, and in particular, to a wearable device.

### BACKGROUND

With the development of society, people are paying more attention to their health as well as their body shape and posture. The size of the legs (e.g., a leg circumference) is one aspect of the body shape that people are particularly concerned about. Therefore, how to allow people to more conveniently monitor their leg circumference during fitness, weight loss, rehabilitation, and other processes is a technical problem that needs to be solved urgently in this field.

### SUMMARY

One or more embodiments of the present disclosure provide a wearable device comprising pants and a sensor assembly fixed on the pants. The sensor assembly includes at least one circumferential strain sensor, the at least one circumferential strain sensor extends along a circumferential direction of a pant leg of the pants and is configured to measure a circumferential dimension of a leg of a user.

In some embodiments, the at least one circumferential strain sensor includes a first strain sensor and a second strain sensor. The first strain sensor is disposed in a thigh region of the pant leg and extends along a circumferential direction of the thigh region to measure a circumferential dimension of a thigh of the user. The second strain sensor is disposed in a calf region of the pant leg and extends along a circumferential direction of the calf region to measure a circumferential dimension of a calf of the user.

In some embodiments, the at least one circumferential strain sensor includes a plurality of first strain sensors spaced apart in a length direction of the pant leg; and/or, the at least one circumferential strain sensor includes a plurality of the second strain sensors spaced apart in the length direction of the pant leg.

In some embodiments, the at least one circumferential strain sensor includes a third strain sensor disposed in a waist region of the pants and extends along a circumferential direction of the waist region to measure a circumferential dimension of the waist of the user.

In some embodiments, the sensor assembly further includes at least one longitudinal strain sensor. The longitudinal strain sensor extends along a length direction of the pant leg, at least a portion of the longitudinal strain sensor is located in the thigh region, and at least another portion of the longitudinal strain sensor is located in the calf region.

In some embodiments, the at least one longitudinal strain sensor and the at least one circumferential strain sensor are located on a same bearing layer, and the at least one longitudinal strain sensor and the at least one circumferential strain sensor are arranged adjacent to each other.

In some embodiments, a first gap is formed between two ends of the first strain sensor, a second gap is formed between two ends of the second strain sensor, and the longitudinal strain sensor passes through the first gap and the second gap.

In some embodiments, the pants include at least two bearing layers stacked in a thickness direction of the pants, and the at least one circumferential strain sensor and the at least one longitudinal strain sensor are disposed on different bearing layers of the at least two bearing layers.

In some embodiments, the at least one longitudinal strain sensor includes a plurality of longitudinal strain sensors, the plurality of longitudinal strain sensors are spaced apart along the circumferential direction of the pant leg.

In some embodiments, the at least one longitudinal strain sensor is located in a region of the pant leg that faces towards or is away from the other pant leg of the pants.

In some embodiments, an elasticity coefficient of the calf region of the pant leg in which the second strain sensor is located is greater than an elasticity coefficient of the thigh region of the pant leg in which the first strain sensor is located.

In some embodiments, the sensor assembly further includes a first electrode module integrated in the pants. The first electrode module includes at least two first electrodes in contact with a skin of the user. The at least two first electrodes are spaced apart along a muscle fiber direction of a same muscle of the leg of the user. The first electrode module is configured to collect an electromyographic (EMG) signal of the muscle of the leg of the user.

In some embodiments, the at least one circumferential strain sensor is arranged between the at least two first electrodes of the first electrode module.

In some embodiments, the wearable device further comprises a processing circuit, wherein the processing circuit is integrated in a waist region of the pants, and the processing circuit is configured to process or send the circumferential dimension of the leg of the user.

One or more embodiments of the present disclosure provide a wearable device comprising pants and a sensor assembly pants fixed on the pants. The sensor assembly includes a second electrode module, the second electrode module includes at least one second electrode, the at least one second electrode extends along a circumferential direction of a pant leg of the pants, and the second electrode module is configured to measure a circumferential dimension of a leg of a user.

In some embodiments, the at least one second electrode includes at least two second electrodes in contact with a skin of the user, and the at least two second electrodes are spaced apart along a muscle fiber direction of a same muscle of the leg of the user. The second electrode module is configured to collect an EMG signal of the muscle of the leg of the user.

In some embodiments, each of the at least two second electrodes is provided with a first signal lead-out end, a second signal lead-out end, and a third signal lead-out end. The first signal lead-out end and the second signal lead-out end are configured to lead out a signal of the circumferential dimension of the leg of the user. The first signal lead-out end and the second signal lead-out end are located at two ends of an extension direction of the second electrode, respectively. The third signal lead-out end is configured to lead out a potential signal.

In some embodiments, the second electrode is configured as a unidirectional deformation electrode or a unidirectional strain-sensitive electrode adapted to a circumferential deformation of the pant leg.

In some embodiments, the sensor assembly further includes a third electrode module. The third electrode module includes at least one third electrode, and the third electrode is configured as a unidirectional deformation electrode or unidirectional strain-sensitive electrode adapted to a length direction of the pant leg.

One or more embodiments of the present disclosure provide a wearable device comprising pants, a sensor assembly fixed on the pants, and a processor. The sensor assembly includes at least one circumferential strain sensor. The at least one circumferential strain sensor extends along a circumferential direction of a pant leg of the pants and is configured to measure a circumferential dimension of a leg of a user. The processor is configured to generate circumference information of the leg of the user by obtaining the circumferential dimension of the leg of the user.

In some embodiments, the circumference information includes circumference change information in a first preset time period. To generate the circumference information of the leg of the user by obtaining the circumferential dimension of the leg of the user, the processor is configured to monitor the circumferential dimension of the leg of the user in the first preset time period to generate the circumference change information.

In some embodiments, the sensor assembly further includes at least one longitudinal strain sensor. The at least one longitudinal strain sensor extends along a length direction of the pant leg and is configured to detect at least a shape of the leg of the user. The processor is configured to generate a model of the leg of the user based on the shape and the circumferential dimension of the leg of the user.

In some embodiments, the processor is configured to generate a first training guidance recommendation based on the circumferential dimension of the leg of the user.

In some embodiments, the wearable device further comprises a first electrode module integrated in the pants. The first electrode module includes at least two first electrodes spaced apart along a muscle fiber direction of a same muscle of the leg of the user. The first electrode module is configured to collect an EMG signal of the muscle of the leg of the user. The processor is configured to generate a second training guidance recommendation based on the circumference change information of the leg of the user and the EMG signal.

In some embodiments, the processing circuit is configured to determine posture information reflecting a posture of the leg of the user based on the shape of the leg of the user and/or the circumferential dimension of the leg of the user. The posture information includes a current posture of the leg of the user and a duration for which the leg of the user is in the current posture.

In some embodiments, the posture includes at least a preset improper posture. To determine the posture information reflecting the posture of the leg of the user based on the shape of the leg of the user and/or the circumferential dimension of the leg of the user, the processor is configured to: in response to determining that the current posture of the leg of the user is the preset improper posture, determine the duration for which the leg of the user is in the preset improper posture based on the shape of the leg of the user and/or the circumferential dimension of the leg of the user; and generate a corrective recommendation based on the duration for which the leg of the user is in the preset improper posture.

In some embodiments, the posture includes at least a walking posture and a running posture, and the processor is configured to: in response to determining that the current posture of the leg of the user is the walking posture or the running posture, determine at least one of a step count, a step length, or a step frequency of the user based on the shape of the leg of the user and/or the circumferential dimension of the leg of the user.

In some embodiments, the posture includes at least a workout posture, and the processor is configured to: in response to determining that the current posture of the leg of the user is the workout posture, count workout movements of the leg of the user based on the shape of the leg of the user and/or the circumferential dimension of the leg of the user.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram of a system for monitoring a leg circumference according to some embodiments of the present disclosure;
FIG. 2 A is a block diagram of an exemplary wearable device according to some embodiments of the present disclosure;
FIG. 2 B is a block diagram of an exemplary wearable device according to some embodiments of the present disclosure;
FIG. 3 is a schematic diagram of an exemplary structure of a wearable device according to some embodiments of the present disclosure;
FIG. 4 is a schematic diagram of an exemplary structure of a wearable device according to some other embodiments of the present disclosure;
FIG. 5 is a schematic diagram of an exemplary structure of a wearable device according to yet some more embodiments of the present disclosure;
FIG. 6 is a schematic diagram of an exemplary structure of a wearable device according to some further embodiments of the present disclosure;
FIG. 7 is a schematic diagram of an exemplary structure of a wearable device according to some other embodiments of the present disclosure;
FIG. 8 is a schematic diagram of an exemplary structure of a wearable device according to some other embodiments of the present disclosure;
FIG. 9 is a schematic diagram of an exemplary structure of a first electrode module according to some embodiments of the present disclosure;
FIG. 10 is a schematic diagram of an exemplary structure of a wearable device according to some embodiments of the present disclosure;
FIG. 11 is a schematic diagram of an exemplary structure of a second electrode module according to some embodiments of the present description; and
FIG. 12 is a block diagram of a processor of a wearable device according to some embodiments of the present disclosure.

### DETAILED DESCRIPTION

To more clearly illustrate the technical solutions of the embodiments of the present disclosure, the accompanying drawings to be used in the description of the embodiments will be briefly described below. Obviously, the accompanying drawings in the following description are only some examples or embodiments of the present disclosure, and that the present disclosure may be applied to other similar scenarios in accordance with these drawings without creative labor for those of ordinary skill in the art. Unless obviously acquired from the context or the context illustrates otherwise, the same numeral in the drawings refers to the same structure or operation.

It should be understood that "system," "device," "unit," and/or "module" as used herein is a way to distinguish between different components, elements, parts, sections, or assemblies at different levels. However, these words may be replaced by other expressions if they accomplish the same purpose.

As indicated in the present disclosure and in the claims, the singular forms "a," "an," and "the" may be intended to include the plural forms as well, unless the context clearly indicates otherwise. In general, the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof.

Flowcharts are used in the present disclosure to illustrate the operations performed by the system according to some embodiments of the present disclosure. It should be understood that the operations described herein are not necessarily executed in a specific order. Instead, the operations may be executed in reverse order or simultaneously. Additionally, one or more other operations may be added to these processes, or one or more operations may be removed from these processes.

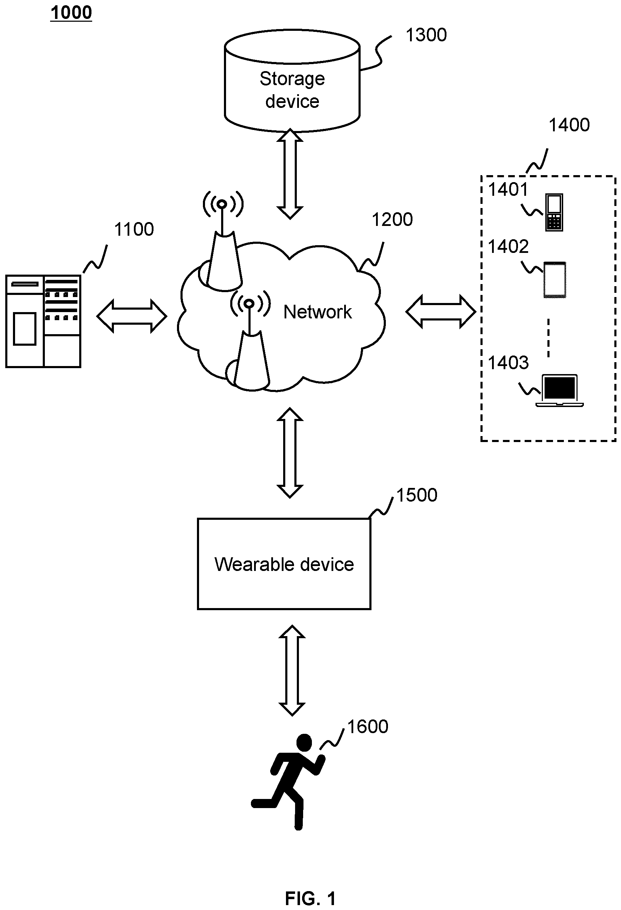

FIG. 1 is a schematic diagram of a system for monitoring a leg circumference according to some embodiments of the present disclosure. Referring to FIG. 1, in some embodiments, a system 1000 for monitoring a leg circumference (hereinafter referred to as the system 1000) may include a processing device 1100, a network 1200, a storage device 1300, one or more terminal devices 1400, and a wearable device 1500. Components of the system 1000 may be connected in a variety of ways. For example, the wearable device 1500 may be connected to the storage device 1300 and/or the processing device 1100 via the network 1200, and the wearable device 1500 may be connected to the storage device 1300 and/or the processing device 1100 directly. As another example, the storage device 1300 may be directly connected to the processing device 1100 or connected to the processing device 1100 via the network 1200. As yet another example, the one or more terminal devices 1400 may be connected to the storage device 1300 and/or the processing device 1100 via the network 1200, and the one or more terminal devices 1400 may be connected to the storage device 1300 and/or the processing device 1100 directly.

In some embodiments, the system 1000 may measure a circumferential dimension of a leg of a user and determine the circumference information of the leg of the user through one or more devices disclosed in the present disclosure. For example, a sensor assembly of the wearable device 1500 measures the circumferential dimension of the leg of the user. As another example, the processing device 1100 may process the circumferential dimension of the leg of the user to determine the circumference information of the leg of the user.

The processing device 1100 may process data and/or information obtained from the wearable device 1500, the storage device 1300, the one or more terminal devices 1400, and/or other components of the system 1000. In some embodiments, the processing device 1100 may obtain circumferential dimensions of the leg (e.g., a thigh, a calf, etc.) of the user 1600 from any one of the wearable device 1500, the storage device 1300, the one or more terminal devices 1400, or a combination thereof and process the circumferential dimension to generate the circumference information of the leg of the user. In some embodiments, the processing device 1100 may obtain a pre-stored computer instruction from the storage device 1300 and execute the computer instruction to implement the system 100.

In some embodiments, the processing device 1100 may be a single server or a group of servers. The group of servers may be centralized or distributed. In some embodiments, the processing device 1100 may be local or remote. For example, the processing device 1100 may access information and/or data from the wearable device 1500, the storage device 1300, and/or the one or more terminal devices 1400 via the network 1200. As another example, the processing device 1100 may be directly connected to the wearable device 1500, the storage device 1300, and/or the one or more terminal devices 1400 to access the information and/or data. In some embodiments, the processing device 1100 may be implemented on a cloud platform. For example, the cloud platform may include a private cloud, a public cloud, a hybrid cloud, a community cloud, a distributed cloud, an inter-cloud cloud, a multi-cloud, and the like, or any combination thereof.

The network 1200 may connect various components of the system 1000 and/or connect the system 1000 to an external resource portion. The network 1200 may enable communication between the components of the system 1000 and communication between the system 1000 and the external resource portion, to facilitate the exchange of data and/or information. For example, the processing device 1100 may obtain the dimensional information (e.g., the circumferential dimension) from the wearable device 1500 and/or the storage device 1300 via the network 1200. As another example, the processing device 1100 may obtain a user operation instruction from the one or more terminal devices 1400 via the network 1200, and exemplary operation instructions may include, but are not limited to, setting user information (e.g., gender, age, height, weight, history of disease, etc.), starting to measure leg circumference, etc.

In some embodiments, the network 1200 may be any form of wired or wireless network, or any combination thereof. Merely by way of example, the network 120 may include a cable network, a wired network, a fiber optic network, a telecommunication network, an intranet, the Internet, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, etc., or any combination thereof. In some embodiments, the network 1200 may include at least one network access point, and at least one component of the system 100 may be connected to the network 1200 via the at least one access point to exchange data and/or information. For example, data such as the circumferential dimension of the leg of the user, a circumferential dimension of the waist of the user, an electromyographic (EMG) signal of a muscle of the leg of the user, and the shape of the leg of the user may be communicated via the network 120.

The storage device 1300 may store data, instructions, and/or any other information. In some embodiments, the storage device 1300 may store data obtained from the wearable device 1500, the processing device 1100, and/or the one or more terminal devices 1400. For example, the storage device 1300 may store the circumferential dimension acquired by the wearable device 1500. In some embodiments, the storage device 1300 may store data and/or instructions used by the processing device 1100 to execute the exemplary methods described in the present disclosure. In some embodiments, the storage device 1300 may include mass memory, removable memory, volatile read-write memory, read-only memory (ROM), or the like, or any combination thereof. Exemplary mass memory may include a disk, an optical disk, a solid-state disk, or the like. In some embodiments, the storage device 1300 may be implemented on a cloud platform. Merely by way of example, the cloud platform may include a private cloud, a public cloud, a hybrid cloud, a community cloud, a distributed cloud, an on-premises cloud, a multi-tiered cloud, etc., or any combination thereof.

In some embodiments, the storage device 1300 may be connected to the network 1200 to communicate with at least one other component of the system 1000 (e.g., the wearable device 1500, the processing device 1100, the one or more terminal devices 1400). At least one component of the system 1000 may access data, instructions, or other information stored in the storage device 1300 via the network 1200. In some embodiments, the storage device 1300 may be directly connected or in communication with one or more components (e.g., wearable device 1500, one or more terminal devices 1400) of the system 1000. In some embodiments, the storage device 1300 may be part of the wearable device 1500 and/or the processing device 1100.

The one or more terminal devices 1400 may receive, send, and/or display data. The received data may include data collected by the wearable device 15000, data stored by the storage device 1300, data generated by the processing device 1100, or the like. For example, the data received and/or displayed by the one or more terminal devices 1400 may include the circumferential dimension collected by the wearable device 1500, the circumference information generated by the processing device 1100 by processing the circumferential dimension, or the like. The sent data may include user input data, instructions, etc. For example, the one or more terminal devices 1400 may send an operation instruction inputted by the user to the wearable device 1500 via the network 1200 to control the wearable device 1500 to perform corresponding data collection.

In some embodiments, the one or more terminal devices 1400 may include a mobile device 1401, a tablet computer 1402, a laptop computer 1403, etc., or any combination thereof. For example, the mobile device 1410 may include a cell phone, a personal digital assistant (PDA), a medical mobile terminal, etc., or any combination thereof. In some embodiments, the one or more terminal devices 1400 may include an input device (e.g., a keyboard, a touch screen), an output device (e.g., a display, a speaker), or the like. In some embodiments, the processing device 1100 may be part of the one or more terminal devices 1400.

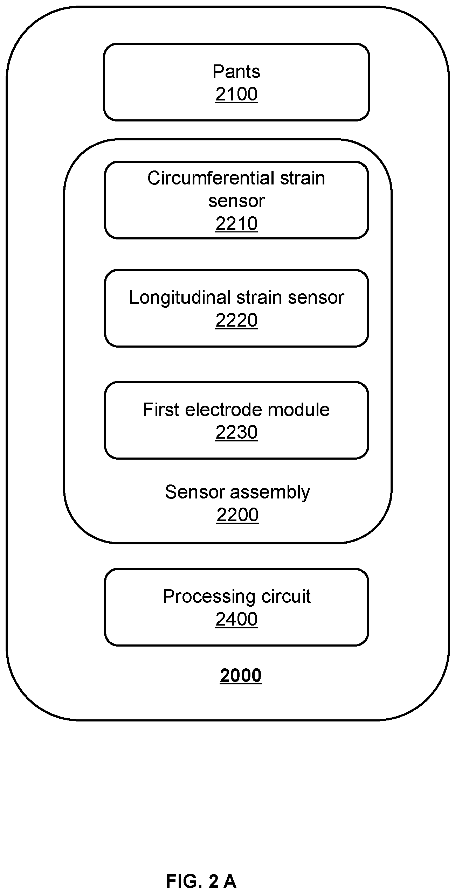

The wearable device 1500 is a device that performs data collection on the user 1600. The data includes data of the leg of the user 1600. Exemplary data may include circumferential dimensions, leg shape, EMG signals, or the like. In some embodiments, the wearable device 1500 may have an independent power source and may send the collected data to other components (e.g., the processing device 1100, the storage device 1300, the one or more terminal devices 1400) in a wired or wirelessly (e.g., Bluetooth, WiFi, etc.) manner. In some embodiments, one or more components on the system 1000 may be implemented on the wearable device 1500. For example, the wearable device 1500 may include the processing device 1100, the storage device 1300, or the like. More descriptions of exemplary structures of the wearable device 150 may be found in FIG.2 A-FIG.10 and the related descriptions thereof.

The user 1600 refers to a user of the wearable device 1500. For example, the user 1600 may be a person concerned about the circumference of his or her legs, an exercise person, a tester of exercise equipment, a patient in need of rehab, or the like. In some embodiments, the user 1600 may issue a query request. For example, the user 1600 inquires about circumference information, circumference change information, stance information, training instruction advice, corrective advice, or the like, for his or her leg. In some embodiments, the user may receive feedback from the wearable device 1500 or the one or more terminal devices 1400, such as receiving a query result (e.g., circumference information, circumference change information, posture information, etc.), a suggestion (e.g., a training instruction suggestion, a correction suggestion, etc.), or the like.

It should be noted that the foregoing descriptions of the system 1000 are intended to be exemplary and illustrative only and do not limit the scope of application of the present disclosure. For those skilled in the art, various modifications and changes may be made to the system 1000 under the guidance of the present disclosure. However, these modifications and changes remain within the scope of the present disclosure.

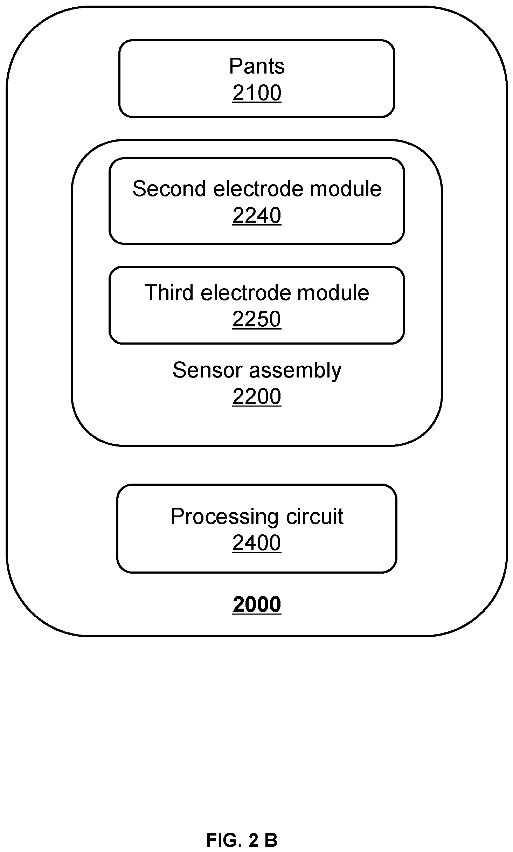

FIG. 2 A and FIG. 2 B are block diagrams of a wearable device 2000 according to some embodiments of the present disclosure. As shown in FIG. 2 A, the wearable device 2000 includes pants 2100 and a sensor assembly 2200 fixed on the pants 2100. The sensor assembly 2200 includes at least one circumferential strain sensor 2210, the at least one circumferential strain sensor 2210 extending along a circumferential direction of a pant leg of the pants 2100 to measure a circumferential dimension of a leg of a user.

The pants 2100 may be of a variety of lengths, e.g., the pants 2100 may be shorts, seven-quarter pants, nine-quarter pants, pants, etc. Specifically, the length of the pants 2100 may be determined based on the position of the circumferential dimension of the leg that the user needs to monitor. For example, when the user only needs to monitor a circumferential dimension of a thigh, the pants 2100 may be shorts with the legs over the thighs, seven-quarter pants, or pants 2100 with a longer length. As another example, when the user needs to monitor a circumferential dimension of a calf, the pants 2100 may be nine-quarter pants or long pants. As yet another example, when the user needs to monitor a circumferential dimension of an ankle, the pants 2100 may be long pants. The pants 2100 is a carrier for carrying a component such as the sensor assembly 2200. In some other embodiments, the pants 2100 may be replaced with other devices capable of being worn on the legs, such as leg straps (e.g., full leg straps, thigh straps, calf straps, etc.), leggings (e.g., full leggings, thighgings, calfgings, etc.), socks (e.g., calf socks, thigh socks, pantyhose, etc.), or the like.

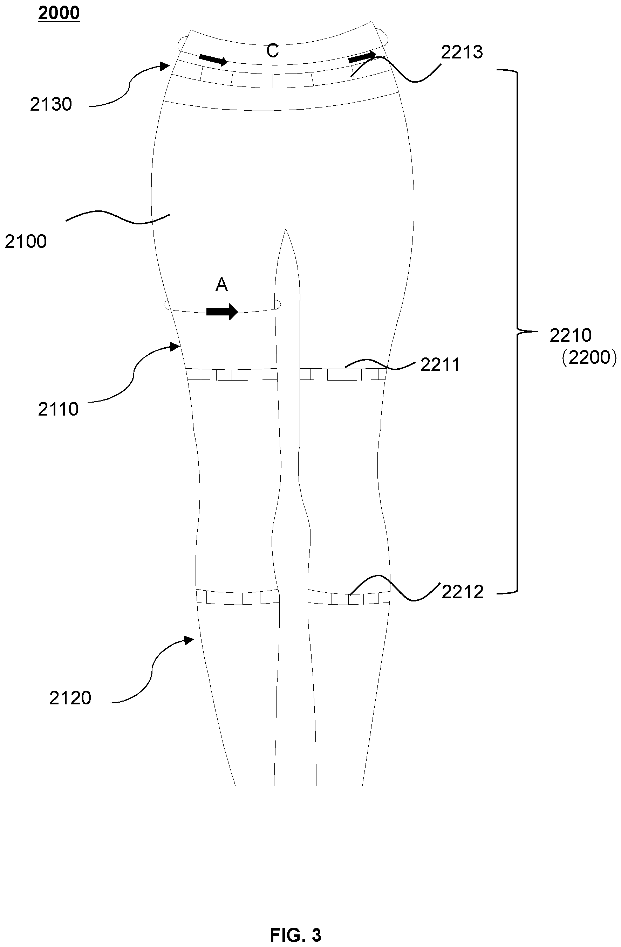

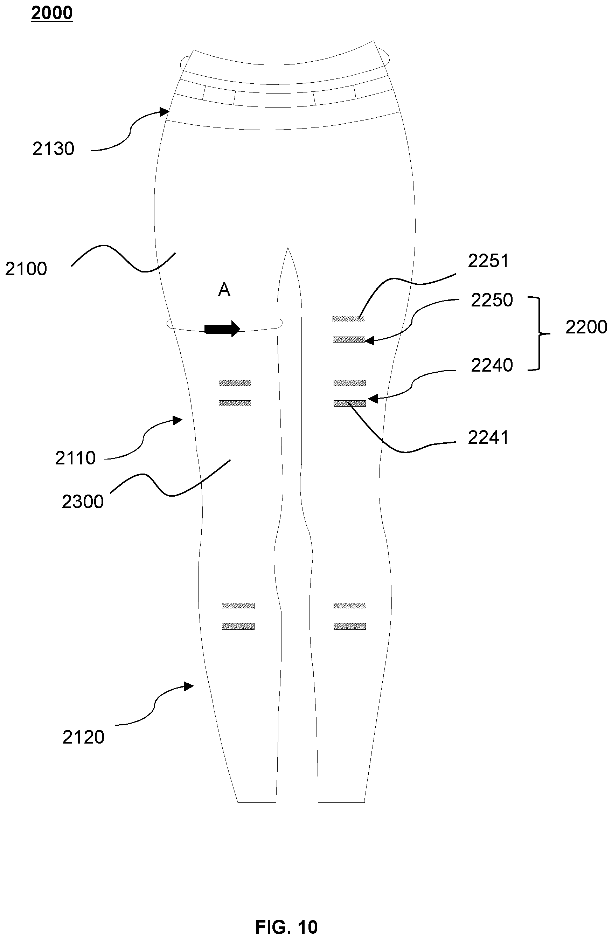

The sensor assembly 2200 is configured to collect data from the leg of the user. In some embodiments, the sensor assembly 2200 may include at least one strain sensor (e.g., the circumferential strain sensor 2210 and a longitudinal strain sensor 2220 in the present disclosure, as shown in FIG. 2A), and the sensor assembly 2200 may measure the circumferential dimension of the leg of the user via the at least one strain sensor (see FIGs. 3-8 and their related descriptions). A strain sensor is a type of sensor that measures the deformation of an object. Because the strain sensor is fixed to the pants 2100, when the user wears the pants 2100, the pants 2100 may deform, and the strain sensor may generate a strain based on the deformation of the pants 2100, thereby generating an electrical signal. In some embodiments, the strain sensor may be flexible, not affecting the fit of the pants 2100 to the leg of the user when the pants 2100 deform. The strain sensor may be a capacitive strain sensor or a resistive strain sensor. It should be noted that each strain sensor (e.g., each first strain sensor 2211/each second strain sensor 2212/each third strain sensor 2213) may include a plurality of sensing modules. The plurality of sensing modules are sequentially arranged along an extension direction of the strain sensors, and each of the sensing modules is configured to experience a different magnitude of strain, thereby generating a different magnitude of electrical signal. For further descriptions of the sensing modules, please refer to the description below regarding the longitudinal strain sensor 2220. In some embodiments, the sensor assembly 2200 may include an electrode module (e.g., a second electrode module 2240 in the present disclosure, as shown in FIG. 2B), and the sensor assembly 2200 may measure the dimensions of the leg of the user via the electrode module (see FIG. 10 and its related description).

In some embodiments, the sensor assembly 2200 may be secured to an inner surface of the pants 2100. In other embodiments, the sensor assembly 2200 may be secured to an outer surface of the pants 2100 to prevent discomfort caused by the sensor assembly 2200 to the user. In yet other embodiments, the pants 2100 may have an interlayer, and the sensor assembly 2200 may be secured to the interlayer of the pants 2100, which may ensure the wearing comfort of the user while protecting the sensor assembly 2200. The sensor assembly 2200 may be secured to the pants through pasting, weaving, or the like.

When the sensor assembly 2200 includes a strain sensor, as shown in FIG. 2 A, the sensor assembly 2200 may include a circumferential strain sensor 2210. The circumferential strain sensor 2210 extends along a circumferential direction of the pant leg of the pants 2100. In the present disclosure, the circumferential direction of the pant leg is indicated by the arrow A in FIG.3 - FIG.8 and FIG. 10, and when the user wears the pants 2100, the circumferential direction of the pant leg is substantially the same as a circumferential direction of the leg of the user. The circumferential strain sensor 2210 may have a strip structure, and the strain sensor of the strip structure may be configured to be arranged circumferentially along (i.e., around) the leg of the user. By configuring the circumferential strain sensor 2210 to extend along the circumferential direction of the pant leg, when the user wears the pants 2100 and the pants 2100 deform along the circumferential direction of the pant leg (e.g., when the pants 2100 are stretched along the circumferential direction), the circumferential strain sensor 2210 may generate strain along the circumferential direction of the pant leg to measure the circumferential dimension of the leg of the user. It may be understood that during wearing, the circumferential strain sensor 2210 may continuously measure, measure at intervals (e.g., once every preset period), or measure according to an instruction, the circumferential dimension of the leg of the user to monitor the circumference of the leg of the user.

In some embodiments, a length of the circumferential strain sensor 2210 may be equal to a circumferential length of the pant leg at which the circumferential strain sensor 2210 is disposed. In some embodiments, the length of the circumferential strain sensor 2210 may be less than the circumferential length of the pant leg at which the circumferential strain sensor 2210 is disposed. For example, the length of the circumferential strain sensor 2210 may be 1/2, 1/3, 1/5, or the like, of the circumferential length of the pant leg at which the circumferential strain sensor 2210 is disposed.

FIG. 3 is a schematic diagram of an exemplary structure of a wearable device according to some embodiments of the present disclosure. In some embodiments, at least one circumferential strain sensor 2210 of the wearable device 2000 may include a first strain sensor 2211 as shown in FIG. 3. The first strain sensor 2211 may be configured to measure a circumferential dimension of a thigh of a user. The first strain sensor 2211 is disposed in a thigh region 2110 of a pant leg and extends in a circumferential direction (i.e., in the direction indicated by arrow A in FIG. 3) along the thigh region 2110 to measure the circumferential dimension of the thigh of the user. Merely by way of example, the first strain sensor 2211 may be a band that extends along the circumference of the thigh region 2110 of the pant leg.

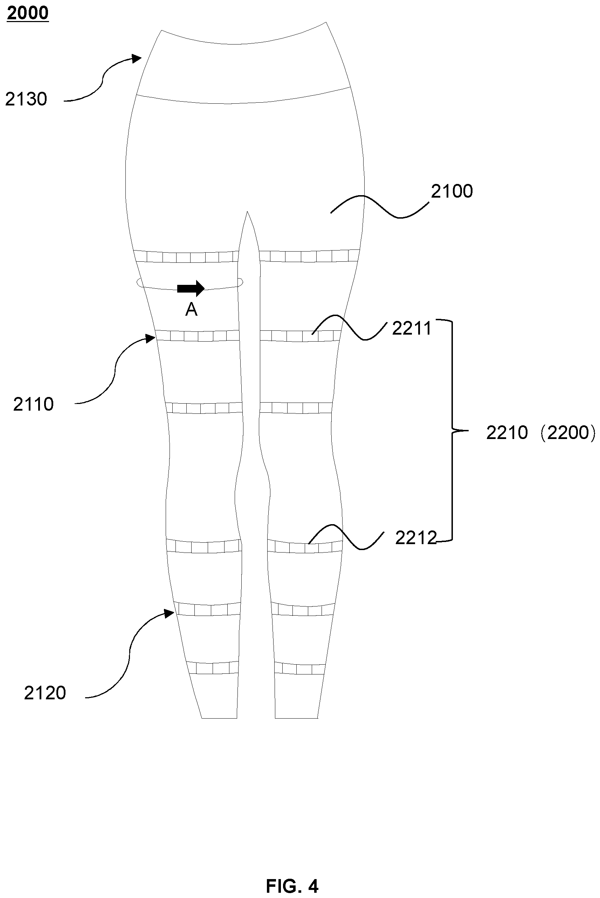

In some embodiments, the at least one circumferential strain sensor 2210 may include a plurality of first strain sensors 2211, the plurality of first strain sensors 2211 being spaced apart along a length direction of the pant leg. For example, the plurality of first strain sensors 2211 may be spaced apart in the thigh region 2110 along the length direction of the pant leg, as shown in FIG. 4. In some embodiments, the plurality of first strain sensors 2211 may be spaced apart at equal intervals. In other embodiments, a distance between two adjacent first strain sensors 2211 may be set according to a preset distance. For example, the preset distance may be 8 cm, 10 cm, etc. The plurality of first strain sensors 2211 are configured such that circumferential dimensions at different positions of the thigh (e.g., at different positions in a length direction of the thigh) may be measured, thereby measuring the circumferential dimensions of the thigh more comprehensively. In some embodiments, each of the plurality of first strain sensors 2211 may be arranged at a particular position on the thigh of the user. For example, a first strain sensor 2211 may be arranged at a position with a maximum circumferential dimension on the thigh and a first strain sensor 2211 may be arranged at a position with a minimum circumferential dimension on the thigh. It should be noted that the position on the thigh with the maximum circumferential dimension and the position on the thigh with the minimum circumferential dimension may be set according to a height or a leg length of the human body, or may be personalized according to a leg shape of the user. In some embodiments, the plurality of first strain sensors 2211 may be arranged along the length direction of the thigh, from top to bottom, at 20% of a thigh length (i.e., a distance from the hip to the knee), 50% of the thigh length, and 80% of the thigh length, respectively.

In some embodiments, the at least one circumferential strain sensor 2210 of the wearable device 2000 may include a second strain sensor 2212, as illustrated in FIG. 3. The second strain sensor 2212 may be configured to measure a circumferential dimension of a calf of the user. In some embodiments, the second strain sensor 2212 is disposed in a calf region 2120 of the pant leg and extends in a circumferential direction (i.e., in the direction indicated by arrow A in FIG. 3) along the calf region 2120 to measure the circumferential dimension of the calf of the user. Merely by way of example, the second strain sensor 2212 may be a band that extends along the circumferential direction of the calf region 2120 of the pant leg.

In some embodiments, the at least one circumferential strain sensor 2210 may include a plurality of second strain sensors 2212, and the plurality of second strain sensors 2212 may be spaced apart along the length direction of the pant leg. For example, the plurality of second strain sensors 2212 may be spaced apart in the calf region 2120 along the length direction of the pant leg, as shown in FIG. 4. In some embodiments, the plurality of second strain sensors 2212 may be spaced apart at equal intervals. In other embodiments, a distance between two adjacent second strain sensors 2212 may be set according to a preset distance. For example, the preset distance may be 8 cm, 10 cm, etc. The plurality of second strain sensors 2212 are configured such that the circumferential dimensions at different positions of the calf (e.g., at different positions in a length direction of the calf) may be measured, so as to more comprehensively measure the circumferential dimensions of the calf. In some embodiments, each of the plurality of second strain sensors 2212 may be arranged at a particular position on the calf of the user. For example, a second strain sensor 2212 may be arranged at a position with a maximum circumferential dimension on the calf and a second strain sensor 2212 may be arranged at a position with a minimum circumferential dimension on the calf. In some embodiments, the plurality of second strain sensors 2212 may be arranged along the length direction of the calf, from top to bottom, at 20% of a calf length (a distance from the knee to the ankle), 50% of the calf length, and 80% of the calf length, respectively.

In some embodiments, as shown in FIG. 3, the at least one circumferential strain sensor 2210 further includes a third strain sensor 2213, the third strain sensor 2213 being disposed in a waist region 2130 of the pants 2100 and extending along a circumferential direction (i.e., in the direction indicated by the arrow C in FIG. 3) of the waist region 2130 to measure a circumferential dimension (i.e., a waist circumference) of the waist of the user. Merely by way of example, the third strain sensor 2213 may be a band-like structure that extends along the circumference direction (which corresponds to a circumference direction of the waist of the user after the user wears the wearable devices 2000) of the waist region 2130 of the pants 2100.

In some embodiments, the at least one circumferential strain sensor 2210 may include a plurality of third strain sensors 2213, the plurality of third strain sensors 2213 being spaced apart along the length direction of the pants 2100. In some embodiments, the plurality of third strain sensors 2213 may be spaced apart at equal intervals. In other embodiments, a distance between two adjacent third strain sensors 2213 may be set according to a preset distance. For example, the preset distance may be 5 cm, 7 cm, etc. The plurality of third strain sensors 2213 may be configured such that circumferential dimensions at different positions of the waist (e.g., different positions in the length direction of the waist) may be measured, thereby measuring the circumferential dimensions of the waist more comprehensively. In some embodiments, each of the plurality of third strain sensor 2213 may be arranged at a particular position on the waist of the user. For example, a third strain sensor 2213 may be arranged at a position with a maximum circumferential dimension on the waist and a third strain sensor 2213 may be arranged at a position with a minimum circumferential dimension on the waist.

A plurality of circumferential strain sensors 2210 (e.g., a first strain sensor 2211, a second strain sensor 2212, and a third strain sensor 2213) located at different positions on the pants 2100 are provided such that the circumferential dimensions of the leg and the waist of the user can be conveniently measured, thereby facilitating the user to monitor his or her leg circumference and waist circumference.

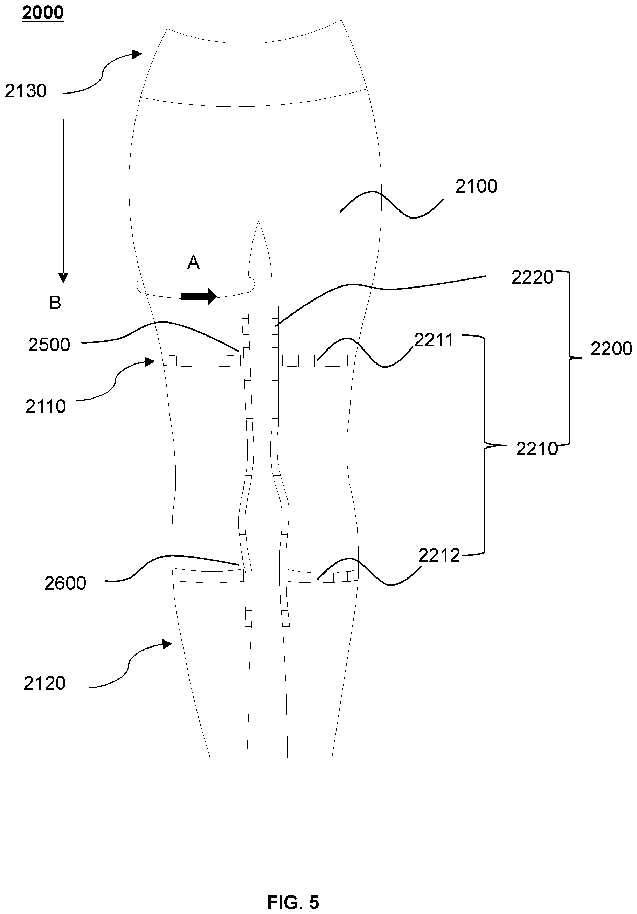

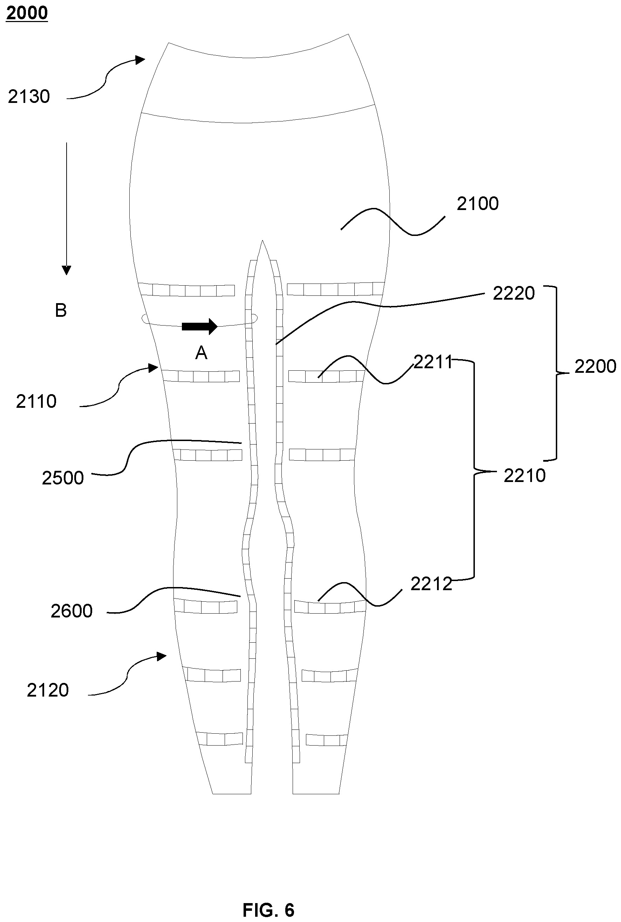

FIG. 5 is a schematic diagram of an exemplary structure of a wearable device according to yet some more embodiments of the present disclosure. FIG. 6 is a schematic diagram of an exemplary structure of a wearable device according to some further embodiments of the present disclosure. In some embodiments, as shown in FIG.2 A, FIG.5, and FIG.6, the wearable device 2000 further includes a longitudinal strain sensor 2220. FIG. 5 illustrates an exemplary arrangement of the longitudinal strain sensor 2220 when one first strain sensor 2211 and one second strain sensor 2212 are provided in the thigh region 2110 and the calf region 2120, respectively. FIG. 6 illustrates an exemplary arrangement of the longitudinal strain sensor 2220 when there are a plurality (three in the figure) of first strain sensors 2211 and a plurality (three in the figure) of second strain sensors 2212. More descriptions of the arrangements of the longitudinal strain sensor 2220 can be found in the following descriptions.

In some embodiments, the longitudinal strain sensor 2220 may be configured to measure a dimension in the length direction of the leg of the user to reflect the shape of the leg of the user. In some embodiments, the longitudinal strain sensor 2220 may extend along the length direction of the pant leg. It should be noted that FIGs. 3-8 may all be regarded as drawings in which the pants 2100 have been worn on the body of the user (merely by way of example, the length direction of the pant leg is substantially the same as the length direction of the leg of the user). Specifically, the length direction of the pants 2100 (or the length direction of the leg of the user) is shown in the direction indicated by arrow B in FIGs. 5-7). It is understood that an extension direction of a portion of the longitudinal strain sensor 2220 may change when the user wears the pants 2100 because the shape of the leg of the user is not a regular cylindrical shape. However, when the user is not wearing the pants 2100, the longitudinal strain sensor 2220 extends in the length direction of the pant leg. At least a portion of the longitudinal strain sensor 2220 is located in the thigh region 2110 and at least another portion of the longitudinal strain sensor 2220 is located in the calf region 2120. In some embodiments, two ends of the longitudinal strain sensor 2220 are located in the thigh region 2110 and the calf region 2120, respectively. In other embodiments, the two ends of the longitudinal strain sensor 2220 are located in the waist region 2130 and the calf region 2120, respectively. As shown in FIG. 5, one end of the longitudinal strain sensor 2220 is located in the thigh region 2110 above a knee region and the other end is located in the calf region 2120 below the knee region. When the user wears the pants 2100, the shape (e.g., an angle between the thigh and the calf, a difference in the circumferential dimension of the thigh and the circumferential dimension of the calf) of the leg of the user affects the deformation of the pant leg in the length direction, which further affects the extension of the longitudinal strain sensor 2220 along the length direction of the pant leg. Based on an electrical signal generated by the longitudinal strain sensor 2220 by detecting the deformation of the pant leg, the shape of the leg, such as a shape characteristic of the leg as a whole, a taper of the thigh, or the like, may be reflected. It may be understood that during wearing, the longitudinal strain sensor 2220 may measure continuously, measure at intervals (e.g., once every preset period), or measure according to an instruction, the dimension of the leg in the length direction of the leg of the user to monitor the shape of the leg of the user.

In some embodiments, the longitudinal sensor 2220 may monitor a posture of the leg of the user by monitoring the shape of the leg of the user. Due to different muscle exertion conditions in various postures of the leg, the shape of the leg of the user may differ. By comparing and analyzing a current shape of the leg of the user with the shape characteristics of the leg of the user in different postures, a current leg posture of the user may be determined. In some embodiments, the longitudinal sensor 2220 may monitor the movement of the leg of the user by detecting the shape of the leg of the user. For example, by continuously measuring or measuring at intervals (e.g., once every preset period) the dimensions of the leg of the user along the length direction of the leg over a time period, one or more leg shapes of the user during the time period may be determined. If only one leg shape is detected during the time period, the user is probably in a roughly stationary state (e.g., sitting, standing, lying down, etc.). If a plurality of leg shapes are detected during the time period, a change (e.g., a periodic change) in the plurality of shapes of the leg may reflect a movement of the user. For example, when the user is performing squatting exercises, the shape of the leg of the user may undergo periodic changes of large-angle bending (i.e., the angle between the thigh and calf decreases substantially, e.g., the angle between the thigh and calf decreases to less than 45°) and straightening over a time period.

In some embodiments, at least one longitudinal strain sensor 2220 is located in a region of the pant leg that faces towards or is away from the other pant leg. The region of the pant leg that faces towards or is away from the other pant leg may be understood as a side of the pant leg. The region of the pant leg that faces towards the other pant leg may be understood as an inner side of the pant leg, and the region of the pant leg that is away from the other pant leg may be understood as an outer side of the pant leg. In such cases, the longitudinal strain sensor 2220 may be configured such that the angle between the thigh and the calf of the user can be more accurately detected, the change in the shape of the leg of the user in the length direction may be more accurately sensed, and whether the user has a common leg shape problem (e.g., X-shaped leg, O-shaped leg, knee hyperextension, etc.) may be determined more accurately.

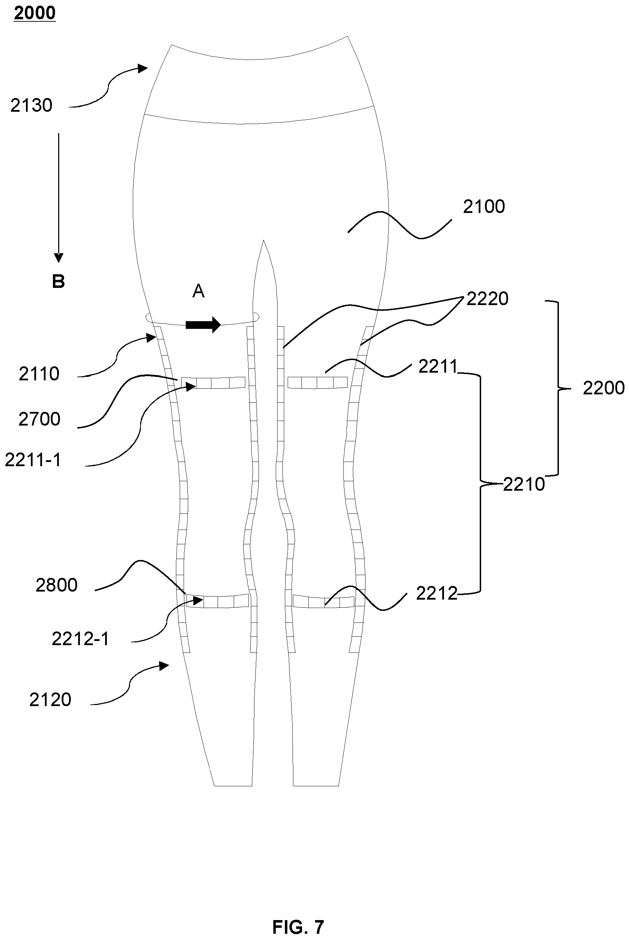

In some embodiments, the at least one longitudinal strain sensor 2220 may include a plurality of longitudinal strain sensors 2220, and the plurality of longitudinal strain sensors 2220 may be spaced apart along the circumferential direction of the pant leg, as shown in FIG. 7. For example, the plurality of longitudinal strain sensors 2220 may be arranged on a front side, a back side, an outer side, an inner side, or the like, of the pants 2100. As another example, when the at least one longitudinal strain sensor 2220 is located at least on the side of the pant leg, at least two longitudinal strain sensing sensors 2220 may be provided on the pant leg, with one of the at least two longitudinal strain sensors arranged on the outer side of the pant leg, and another one of the at least two longitudinal strain sensors 2220 arranged on the inner side of the pant leg. By providing a plurality of strain sensors, the shape of the leg of the user at different positions in the circumferential direction can be sensed more accurately, and thus the overall shape of the leg of the user can be measured more accurately.

In some embodiments, the at least one longitudinal strain sensor 2220 and the at least one circumferential strain sensor 2210 may be located on the same bearing layer of the pants 2100 for ease of processing. In some embodiments, the at least one circumferential strain sensor 2210 (the first strain sensor 2211 and/or the second strain sensor 2212) may be arranged in an overlapping manner with the at least one longitudinal strain sensor 2220. In some embodiments, the at least one longitudinal strain sensor 2220 may be arranged in an adjacent manner with the at least one circumferential strain sensor 2210. Adjacent arrangement may be understood as that there is no overlap between the at least one longitudinal strain sensor 2220 and the at least one circumferential strain sensor 2210 disposed on the same bearing layer in a thickness direction of the pants. The at least one longitudinal strain sensor 2220 and the at least one circumferential strain sensor 2210 located on the same bearing layer may be disposed adjacent to each other or spaced apart by a distance.

In some embodiments, when the first strain sensor 2211 and the second strain sensor 2212 are provided on the same bearing layer of the pants 2100, a first gap 2500 is formed between two ends (e.g., two ends of a band-like structure) of the first strain sensor 2211 and a second gap 2600 is formed between two ends (e.g., two ends of a band-like structure) of the second strain sensor 2212. The first gap 2500 and the second gap 2600 may correspond to each other in the length direction of the pant leg. In other words, both the first gap 2500 and the second gap 2600 are located in a same straight line extending along the length direction of the pant leg. The at least one longitudinal strain sensor 2220 passes through the first gap 2500 and the second gap 2600. As shown in FIG. 5, the first gap 2500 and the second gap 2600 are both located on the inner side of the pant leg, and the longitudinal strain sensor 2220 passes through the first gap 2500 and the second gap 2600. As shown in FIG. 6, when there are a plurality of first strain sensors 2211 and a plurality of second strain sensors 2212, the longitudinal strain sensor 2220 may pass through the first gap 2500 formed by each of the plurality of first strain sensors 2211, respectively, and through the second gap 2600 formed by each of the plurality of second strain sensors 2212, respectively. By setting the first gap 2500 and the second gap 2600, the distribution of the at least one circumferential strain sensor 2210 and the at least one longitudinal strain sensor 2220 on the pants 2100 may be more reasonable to avoid an overlapping portion from generating a large bulge that affects the comfort of the user. Furthermore, the at least one circumferential strain sensor 2210 and the at least one longitudinal strain sensor 2220 can work stably without interfering with each other.

In some embodiments, when the at least one longitudinal strain sensors 2220 includes a plurality of longitudinal strain sensors 2220, the first strain sensor 2211 may include a plurality of first sub-strain sensors 2211-1 spaced apart at intervals along the circumferential direction of the pant leg, and the at least one second strain sensor 2212 may include a plurality of second sub-strain sensors 2212-1 spaced apart at intervals along the circumferential direction of the pant leg. The plurality of first sub-strain sensors 2211-1 and the plurality of second sub-strain sensors 2212-1 may be arranged in a one-to-one correspondence. The longitudinal strain sensor 2220 passes through a gap between two adjacent first sub-strain sensors 2211-1 and a gap between two adjacent second sub-strain sensors 2212-1. For example, the gap between any two adjacent first sub-strain sensors 2211-1 may be a third gap 2700, and the gap between any two adjacent second sub-strain sensors 2212-1 may be a fourth gap 2800. There are a plurality of third gaps 2700 and a plurality of fourth gaps 2800 (e.g., at least two). The plurality of third gaps 2700 and the plurality of fourth gaps 2800 are arranged in a one-to-one correspondence. One third gap 2700 and one fourth gap 2800 arranged in the same straight line extending along the length direction of the pant leg are regarded as being correspondingly arranged. Any of the plurality of longitudinal strain sensors 2220 passes through the correspondingly arranged third gap 2700 and fourth gap 2800. As shown in FIG. 7, the plurality of first sub-strain sensors 2211-1 may be spaced apart along the circumferential direction (around the thigh) of the pant leg. The plurality of second sub-strain sensors 2212-1 may be spaced along the circumferential direction (around the calf) of the pant leg. In FIG. 7, the counts of the first sub-strain sensors 2211-1 and the second sub-strain sensors 2212-1 are both two. The two first sub-strain sensors 2211-1 may be located on the front side and the back side of the pant leg (e.g., the thigh region 2110), respectively (the first sub-strain sensor 2211-1 on the back side is not shown in FIG. 7), and the two second sub-strain sensors 2212-1 may be located on the front and back side of the pant leg (e.g., the calf region 2120), respectively (the first sub-strain sensor 2211-1 on the back side is not shown in FIG. 7). The counts of the third gaps 2700 and the fourth gaps 2800 are both two. The two third gaps 2700 may be located on the inner side and the outer side of the pant leg, respectively. The two fourth gaps 2800 may be located on the inner side and the outer side of the pant leg, respectively, correspondingly arranged with respect to the third gaps 2700. It may be understood that the count, length, and relative position of the first sub-strain sensors 2211-1 relative to each other along the circumferential direction of the thigh region 2110 of the pant leg may be determined based on the circumferential dimension of the thigh and the count of the longitudinal strain sensors 2220. The count, length, and relative position of the second sub-strain sensor 2212-1 along the circumference direction of the calf region 2120 of the pant leg may be determined based on the circumferential dimension of the calf and the count of the longitudinal strain sensors 2220. The relative position of the first sub-strain sensors 2211-1 along the circumferential direction of the thigh region 2110 of the pant leg determines the position of the third gaps 2700, and the relative position of the second sub-strain sensors 2212-1 along the circumferential direction of the calf region 2120 determines the position of the fourth gaps 2800. The relative position of the first sub-strain sensors 2211-1 along the circumferential direction of the thigh region 2110 of the pant leg and the relative position of the second sub-strain sensor 2212-1 along the circumferential direction of the calf region 2120 of the pant leg are configured such that the plurality of third gaps 2700 and the plurality of fourth gaps 2800 are arranged in a one-to-one correspondence. In such cases, even if the count of the longitudinal strain sensors 2220 is large, the longitudinal strain sensors 2220, the first strain sensors 2211, and the second strain sensors 2212 may be reasonable arranged such that the circumferential dimension, shape, and/or length of the leg of the user are measured accurately.

In summary, by forming gaps (e.g., the first gap 2500, the second gap 2600, the third gap 2700, and the fourth gap 2800) on the at least one circumferential strain sensor 2210 to arrange the at least one longitudinal strain sensor 2220, the at least one longitudinal strain sensor 2220 and the at least one circumferential strain sensor 2210 may not interfere with each other while ensuring the reasonable arrangement of the at least one longitudinal strain sensor 2220 and the at least one circumferential strain sensor 2210, and wearing discomfort caused by the overlapping of the at least one longitudinal strain sensor 2220 and the at least one circumferential strain sensor 2210 is avoided.

In some embodiments, the pants 2100 includes at least two bearing layers stacked in a thickness direction of the pants 2100. Specifically, a count of the bearing layers may be two, three, five, etc. The at least one circumferential strain sensor 2210 and the at least one longitudinal strain sensor 2220 are disposed on different bearing layers of the at least two bearing layers. In some embodiments, there may be an overlap between a projection of the circumferential strain sensor 2210 and a projection of the longitudinal strain sensor 2220 located on different bearing layers in space (along the thickness direction of the pants 2100). For example, each of the at least one first strain sensor 2211 and the at least one second strain sensor 2212 may be a complete ring (without a gap), and the longitudinal strain sensor 2220 may extend directly from above the first strain sensor 2211 to below the second strain sensor 2212. In other embodiments, the projections of the circumferential strain sensor 2210 and the longitudinal strain sensor 2220 located on different bearing layers in space (along the thickness direction of the pants 2100) may not overlap. By setting the at least one circumferential strain sensor 2210 and the at least one longitudinal strain sensor 2220 on different bearing layers, even if there is an overlap between the projection of the circumferential strain sensor 2210 and the projection of the longitudinal strain sensor 2220 in space (along the thickness direction of the pants 2100), the circumferential strain sensor 2210 and the longitudinal strain sensor 2220 do not come into direct contact with each other, thereby ensuring stable operation of the circumferential strain sensor and the longitudinal strain sensor.

It should be understood that the configuration manner (e.g., the count and the position) of the strain sensors (e.g., the at least one circumferential strain sensor 2210 and the at least one longitudinal strain sensor 2220) on the left and right pant legs of the pants 2100 may be different, and the specific configuration manner of the strain sensors on the left and right pant legs may be designed according to usage needs of different users. For example, for a user needing single-leg rehab, the strain sensor may be set only on the pant leg corresponding to the leg to be rehabbed.

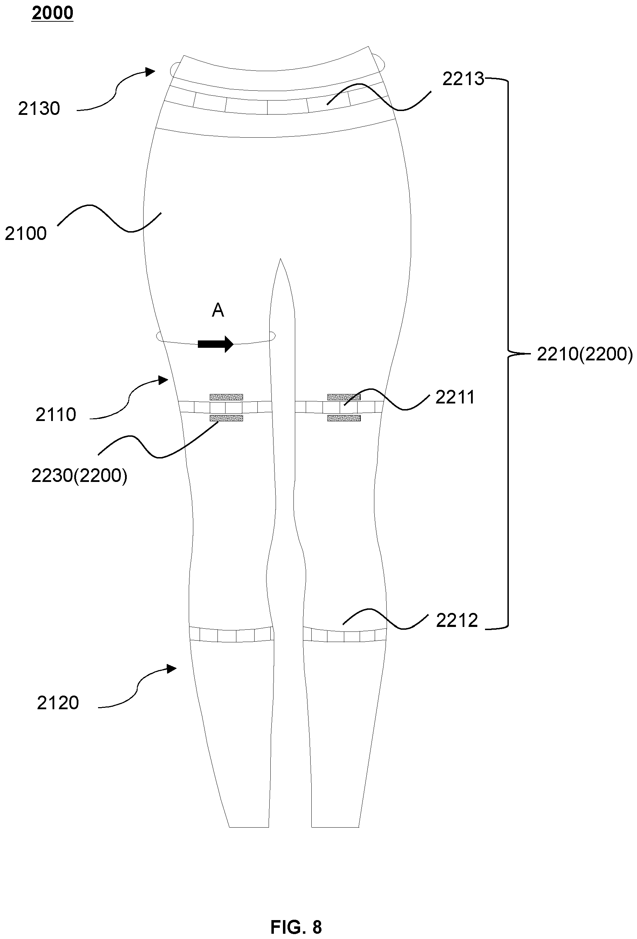

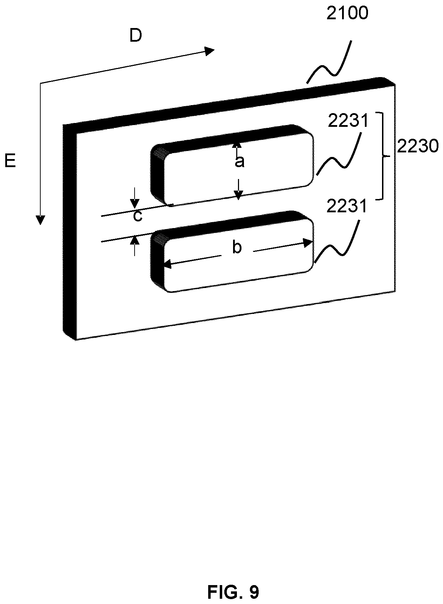

FIG. 8 is a schematic diagram of an exemplary structure of a wearable device according to some other embodiments of the present disclosure. FIG. 9 is a schematic diagram of an exemplary structure of a first electrode module according to some embodiments of the present disclosure. As shown in FIG. 8 and FIG. 9, the wearable device 2000 may further include a first electrode module 2230. The first electrode module 2230 is configured to collect an electromyographic (EMG) signal of a muscle of a leg of a user. The first electrode module 2230 is configured to collect an EMG signal from a muscle of the leg of the user such that the exercise process (e.g., a process of working out, running, walking, etc.) of the user may be monitored based on the EMG signal, and the user may further be provided with exercise feedback. The EMG signal is a type of bioelectrical signal generated along with muscle contraction action. Based on an analysis result of the EMG signal, an activity and a function of the muscle may be reflected.

In some embodiments, the first electrode module 2230 may also be configured to detect a body composition of the leg of the user. For example, the first electrode module 2230 may detect an amount of fat, an amount of bone mass, or the like, in the leg of the user. The first electrode module 2230 can release an electrical signal of one or more frequencies into the human body and then collect the electrical signal. Since electrical signals of different frequencies propagate differently through various types of tissues in the human body, the impedance information may vary accordingly. By analyzing impedance information at different frequencies, the body composition can be interpreted.

The first electrode module 2230 may be integrated in the pants 2100. The first electrode module 2230 may include two first electrodes. The two first electrodes 2231 of the first electrode module 2230 are in contact with the skin of the user to collect EMG signals on a skin surface. The two first electrodes 2231 are spaced apart along a muscle fiber direction (the direction indicated by the arrow E in FIG. 9) of a same muscle of the leg of the user. Arrow D in FIG. 9 illustrates an extension direction of the electrodes. The two first electrodes 2231 may be configured to collect EMG signals of the same muscle of the user. In some embodiments, a differential operation may be performed on the EMG signals collected by different electrodes on the same muscle to reflect a force exertion of the muscle. The same muscle refers to a single muscle or the same muscle group (e.g., the quadriceps muscle group that includes the rectus femoris, lateral femoris, medial femoris, and intermediate femoris).

Merely by way of example, when the wearable device 2000 is worn by the user, the at least two first electrodes 2231 are spaced apart along the muscle fiber direction of the muscle and extend in an extension direction perpendicular to the muscle fiber direction, respectively. Different locations in the muscle fiber direction have different electrical potentials, wherein a location of the muscle fiber where one of the at least two first electrodes 2231 is located has a first electrical potential, and a location of the muscle fiber where another one of the at least two first electrodes 2231 is located has a second potential. There is a potential difference between the first potential and the second potential, and the potential difference may reflect the force exertion of the muscle.

In some embodiments, the signal acquisition accuracy and/or efficiency of the electrodes may be improved by designing dimensions of the first electrodes 2231, a spacing between adjacent first electrodes 2231, or the like, to achieve more accurate and efficient signal acquisition. As shown in FIG. 9, a width of the first electrode 2231 in the muscle fiber direction is denoted as a, a length of the first electrode 2231 in the extension direction is denoted as *b,* and a spacing (i.e., a distance between two adjacent sides of two adjacent first electrodes 2231) between two adjacent first electrodes 2231 is denoted as *c.* In some embodiments, the length *b* of the first electrode 2231 may be determined based on a size (or referred to as a muscle width) of the muscle in the extension direction (the direction indicated by arrow D in FIG. 8) of the electrode. For example, the length *b* may have a relatively large value to minimize noise caused by a relative displacement between the electrode and the muscle, or to minimize an effect on a strength of the collected EMG signal when the electrode is deviated from a position of the muscle. Merely by way of example, the length *b* may be greater than or equal to the muscle width, greater than or equal to 30%, 50%, 60%, 80%, or the like of the muscle width. As another example, if the length *b* is too large, the two first electrodes may span two or more muscle groups (e.g., a target muscle group and a non-target muscle group), causing the collected EMG signals to interfere with each other, and further making it impossible to accurately detect the EMG signals of a target muscle. Thus, the length *b* may not be too large. Merely by way of example, the length *b* may be greater than or equal to 30% of a width of the target muscle and less than or equal to a sum of the width of the target muscle and a spacing between the target muscle and a non-target muscle. As another example, length *b* may be greater than or equal to 50% of the width of the target muscle and less than or equal to the width of the target muscle. In some embodiments, the length *b* of the electrode corresponding to a single target muscle may be determined based on test data for the muscle. Taking the rectus femoris muscle as an example, a range of the length *b* of the electrode corresponding to the rectus femoris muscle may be determined based on the test data of the muscle. For example, the length *b* corresponding to the rectus femoris muscle may be in a range of 1 mm-120 mm. As another example, the length *b* corresponding to the rectus femoris muscle may be in a range of 10 mm-100 mm. As yet another example, the length *b* corresponding to the rectus femoris muscle may be in a range of 20 mm-80 mm. In some embodiments, a scaling factor for a single target muscle may be determined based on a relative relationship (e.g., a size relationship) of the single target muscle to other target muscles, thereby determining the length *b* of the electrode corresponding to each muscle. For different target muscles, different scaling factors may be determined. Merely by way of example, the dimension of the biceps femoris muscle is similar to the rectus femoris muscle, and a scaling factor for the biceps femoris may be in a range of 0.8-1 (e.g., 0.9). For example, the length *b* of the electrode corresponding to the biceps femoris muscle may be in a range of 0.9 mm-108 mm. As another example, the length b of the electrode corresponding to the biceps femoris muscle may be in a range of 9 mm-90 mm. As yet another example, the length b of the electrode corresponding to the biceps femoris muscle may be in a range of 18 mm-72 mm. As another example, the scaling factor for the tensor fasciae lata may be in a range of 0.5-0.8. As yet another example, the scaling factor for the tibialis anterior muscle may be in a range of 0.3-0.7. As a further example, the scaling factor for the gastrocnemius muscle may be in a range of 0.4-0.8.

In some embodiments, the strength of the EMG signal collected by the electrodes may be positively correlated with the spacing *c* between two adjacent first electrodes 2231. The spacing *c* may not be too small so as not to affect the strength of the EMG signal. When the spacing *c* is too large, inconsistency between the two adjacent first electrodes 2231 may be caused, which increases the noise of the EMG signal, and an excessively large spacing c may exceed a muscle range. Therefore, the spacing c of the two adjacent first electrodes 2231 may be set within a preset range. For example, the spacing c may be in a range of 1 mm-50 mm. As another example, the spacing c may be in a range of 5 mm-40 mm. As yet another example, the spacing *c* may be in a range of 10 mm-30 mm.

In some embodiments, to avoid phase cancellation of EMG signals at end positions of the electrode that may result in a reduction in the strength of the EMG signals, the width a of a single electrode and/or the spacing c between adjacent electrodes may be set in a preset range. For example, the end positions may be two ends (i.e., two ends forming the width *a*) of a single electrode along the muscle fiber direction. Correspondingly, the width a of the single electrode may be set within the preset range. As another example, the end positions may be two ends (i.e., two ends of the first electrode 2231 away from each other) of the electrode as a whole in the muscle fiber direction. Correspondingly, the width 2a+c of two adjacent first electrodes 2231 as a whole needs to be set within the preset range. In some embodiments, the preset range may be related to a target frequency (e.g., 0-500 Hz, 10 Hz -300 Hz, 30 Hz -200 Hz, 60 Hz -140 Hz, etc.) at which phase cancellation needs to be avoided. For example, the width a of a single first electrode 2231 may be less than a half-wavelength of the target frequency of the EMG signal. As another example, the width 2a+c of two adjacent first electrodes 2231 as a whole may be less than a full wavelength of the target frequency of the EMG signal.

According to the above description relating to the dimensions of the electrodes, the length b of the first electrode 2231 is relatively large and the width a is relatively small. In some embodiments, the length *b* of the first electrode 2231 in the muscle fiber direction is greater than the width a of the first electrode 2231 in the extension direction (i.e., b/a ≥ 1).

In some embodiments, the performance or comfort of the wearable device 2000 may be improved by designing a material, a structure, or the like of the at least two first electrodes 2231. For example, the at least two first electrodes 2231 may be waterproof, which may prevent short-circuiting between electrodes. As another example, the at least two first electrodes 2231 may be made of an elastic material so that the wearable device 2000 may be put on and taken off more easily, improving the comfort of the wearable device 2000. Merely by way of example, an elasticity of the elastic material of the at least two first electrodes 2231 may be in a range of 0% to 200%. As yet another example, the at least two first electrodes 2231 may be resistant to withstand multiple washing machine loads, repeated donning and doffing, skin rubbing, or the like without affecting other properties. As a further example, the at least two first electrodes 2231 may be corrosion resistant (e.g., sweat corrosion). As a still further example, the at least two first electrodes 2231 may have good electrical conductivity. Merely by way of example, the at least two first electrodes 2231 may have a conductivity of greater than 10K Ω/cm, and a change in conductivity of the at least two first electrodes 2231 under the influence of, for example, deformation of the electrode may be less than 10K Ω or less than 20% of the original conductivity. As a still further example, the at least two first electrodes 2231 may be permeable. Merely by way of example, the at least two first electrodes 2231 may be made of a breathable material (e.g., conductive fabric). As another example, the at least two first electrodes 2231 may have a gas permeable structure (e.g., perforations, intermediate layer gas channels, etc.). As a further example, the at least two first electrodes 2231 may have a degree of softness that ensures comfort of the wearable device 2000. As a further example, the at least two first electrodes 2231 may protrude towards the skin surface with respect to a substrate structure (or a wearing body), so that a localized pressurization effect may be achieved to promote the fit of the at least two first electrodes 2231 to the skin surface. Merely by way of example, a protrusion height of the at least two first electrodes 2231 may be in a range of 0-10 mm. As another example, a filler may be provided between the at least two first electrodes 2231 and the substrate structure to realize the projection of the at least two first electrodes 2231. The filler is a substance (e.g., a sponge, etc.) having a predetermined resilience, softness, water retention capacity, or the like. The predetermined resilience and softness ensure that the wearable device 2000 is comfortable when worn. The predetermined water retention capacity can reduce the accumulation of excessive water on the skin surface and at the same time store part of the water to guarantee the at least two first electrodes 2231 and the skin to have a good contact for a long time.

In some embodiments, the at least one circumferential strain sensor 2210 is arranged between two first electrodes 2231 of the first electrode module 2230 to ensure that the at least one circumferential strain sensor 2210 and the first electrode module 2230 are arranged compactly, thereby avoiding overlapping adversely affects the extensibility of the at least one circumferential strain sensor 2210, while ensuring stable operation of the at least one circumferential strain sensor 2210 and the first electrode module 2230.

In some embodiments, there may be a plurality of first electrode modules 2230, and the plurality of first electrode modules 2230 may be arranged in regions such as the thigh region 2110 and the calf region 2120 of the pant leg. The plurality of first electrode modules 2230 disposed in the thigh region 2110 may be spaced apart along the circumferential direction of the pant leg, and the first strain sensors 2211 may be arranged between two first electrodes 2231 of each first electrode module 2230. FIG. 8 illustrates a case in which the first electrode module 2230 is disposed in the thigh region 2110 and on the front side of the pant leg. In some embodiments, a plurality of first electrode modules 2230 disposed in the calf region 2120 may be spaced apart along the circumferential direction of the pant leg, and the first strain sensors 2211 may be disposed in the calf region 2120 between two first electrodes 2231 of each first electrode module 2230. In some other embodiments, the at least one circumferential strain sensors 2210 may also be disposed above or below the first electrode module 2230.

FIG. 10 is a schematic diagram of an exemplary structure of a wearable device according to some embodiments of the present disclosure. In some embodiments, as shown in FIG. 2B and FIG. 10, the sensor assembly 2200 includes a second electrode module 2240. The second electrode module 2240 includes at least one second electrode 2241. The second electrode module 2240 is arranged on the pants 2100, the at least one second electrode 2241 extends along a circumferential direction of a pant leg of the pants, and the second electrode module 2240 may be configured to measure a circumferential dimension of a leg of the user. When the user wears the pants 2100, the pant leg generates a circumferential deformation, causing the at least one second electrode 2241 to simultaneously deform along the circumferential direction of the pant leg, resulting in a change (e.g., increase) in the resistance of the at least one second electrode 2241. By detecting the change in the resistance of the at least one second electrode 2241, an amount of circumferential deformation of the second electrode 2241 may be determined, and the circumferential dimension of the leg of the user may be determined. By providing the second electrode module to collect the circumferential dimension of the leg of the user, an area occupied by the sensor assembly 2200 on the pants 2100 may be reduced, thereby effectively avoiding wearing discomfort. It may be understood that during wearing, the second electrode module 2240 may measure continuously, measure at intervals (e.g., once every preset period), or measure according to an instruction, the circumferential dimension of the leg of the user to monitor the circumference of the leg of the user.

In some embodiments, the second electrode module 2240 includes at least two second electrodes 2241, and the second electrodes 2241 may be in contact with the skin of the user. The at least two second electrodes 2241 are spaced apart along a muscle fiber direction of the same muscle of the leg of the user. In such cases, the second electrode module 2240 may measure the dimensions of the leg of the user and collect an EMG signal of a muscle of the leg of the user. The principle of the second electrode module 2240 collecting the EMG signal is similar to the principle of the first electrode module collecting the EMG signal, as described above regarding the first electrode module 2230. The at least two second electrodes 2241 is configured to fit the skin of the user such that the second electrode module 2240 monitors the activity and function of the leg of the muscle of the user by collecting the EMG signal of the muscle of the leg of the user while detecting the circumference of the leg by measuring the circumferential dimensions of the leg of the user.

In some embodiments, there may be a plurality of second electrode modules 2240, and the plurality of second electrode modules 2240 may be arranged in regions such as the thigh region 2110 and the calf region 2120 of the pant leg. The plurality of second electrode modules 2240 located in the thigh region 2110 and the plurality of second electrode modules 2240 located in the calf region 2120 may be spaced apart along the circumferential direction of the pant leg. In some embodiments, the plurality of second electrode modules 2240 may also be spaced apart along a length direction of the pant leg. In some embodiments, the plurality of second electrode modules 2240 may also be arranged in the waist region 2130 of the pants to measure the waist circumference of the user.

In some embodiments, the second electrode 2241 may be made of a material that is conductive but also possesses an impedance characteristic. For example, the second electrode 2241 may be made of a material with a conductivity of less than 500 Ω/cm. In some embodiments, the second electrode 2241 may be made of conductive silicon (with a resistance at the kilo-ohm level). The second electrode 2241 is made of a specific material such that the resistance of the second electrode 2241 is increased, and the amount of change in the resistance of the second electrode 2241 affected by the amount of the deformation of the second electrode 2241 is subsequently increased, thereby enhancing the accuracy of the second electrode 2241 in measuring the circumferential dimension of the leg of the user.

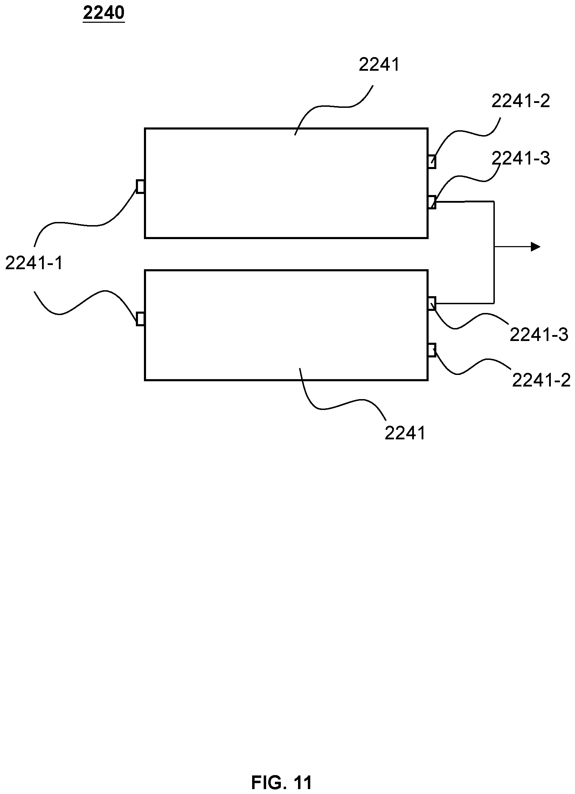

FIG. 11 is a schematic diagram of an exemplary structure of a second electrode module 2240 according to some embodiments of the present disclosure. In some embodiments, the second electrode module 2240 includes at least two second electrodes (FIG. 11 shows two second electrodes). Each second electrode 2241 is provided with a first signal lead-out end 2241-1, a second signal lead-out end 2241-2, and a third signal lead-out end 2241-3. The first signal lead-out end 2241-1 and the second signal lead-out end 2241-2 are configured to lead out a signal of a circumferential dimension of a leg of a user, and the third signal lead-out end 2241-3 is configured to lead out a potential signal. The first signal lead-in end 2241-1 and the second signal lead-out end 2241-2 are located at two ends of an extension direction of the second electrode 2241, respectively, to facilitate measuring a resistance of the second electrode 2241 through the first signal lead-in end 2241-1 and the second signal lead-out end 2241-2. The third signal lead-out end 2241-3 of each second electrode 2241 may lead out the potential signal collected by the second electrode 2241. Based on the potential signals led out by the third signal lead-out ends of at least two second electrodes 2241, a potential difference may be determined, thereby enabling the second electrode module 2240 to collect an EMG signal. The first signal lead-in end 2241-1, the second signal lead-in end 2241-2, and the third signal lead-in end 2241-3 are configured such that the second electrode module 2240 may measure the circumferential dimension of the leg of the user and collect the EMG signal of a muscle of the leg of the user without interfering with each other, and the signal of the circumferential dimension of the leg and the EMG signal can be led out without interfering with each other.

In some embodiments, the second electrode 2241 is a unidirectional deformable electrode adapted to a predetermined direction. A unidirectional deformation electrode that is adapted to a predetermined direction is an electrode that deforms only along a predetermined direction. Thus, even if the unidirectional deformation electrode is subjected to forces from more than one direction, it deforms only along the predetermined direction, with almost no deformation occurring in other directions. For example, when the unidirectional deformation electrode is subjected to forces in more than one direction, an amount of deformation (e.g., an extended length, f1) of the unidirectional deformation electrode in the predetermined direction is much larger than an amount of deformation (e.g., an extended length, f2) of the unidirectional deformation electrode in the other directions (i.e., directions other than the predetermined direction). For example, f1 is more than 10 times of f2.

In some embodiments, the second electrode 2241 may be configured as a unidirectional deformation electrode through a structural design or a weaving form. Merely by way of example, when the second electrode 2241 is a unidirectional deformation electrode provided for circumferential deformation of the pant leg, the second electrode 2241 may include a plurality of electrode sheets arranged along a length direction (perpendicular to a circumferential direction of the pant leg) of the pant leg. An elastic member (e.g. an elastic cord) may be provided between two adjacent electrode sheets. When the pant leg deforms along its length direction, the elastic member preferentially deforms, and the electrode sheets are not prone to deform along the length direction of the pant leg. When the pant leg deforms along its circumferential direction, the electrode sheets undergo strain, resulting in a change in the resistance of each electrode sheet, which in turn changes the resistance of the second electrode 2241.

In other embodiments, the second electrode 2241 is a unidirectional strain-sensitive electrode adapted to a predetermined direction. A unidirectional strain-sensitive electrode adapted to a predetermined direction is an electrode that changes electrical resistance only in response to a deformation of the unidirectional strain-sensitive electrode along the predetermined direction. In other words, the unidirectional strain-sensitive electrode deforms in response to forces applied from various directions, but the resistance of the unidirectional strain-sensitive electrode changes only in response to a deformation occurring in the predetermined direction and does not change (significantly) in response to deformations in other directions. For example, a change in the resistance of the unidirectional strain-sensitive electrode in response to an amount of deformation in the predetermined direction, denoted as e1, is much larger than a change in the resistance of the unidirectional strain-sensitive electrode in response to the same amount of deformation in the other directions (directions other than the predetermined direction), e2. For example, e1 is more than 10 times of e2.

In some embodiments, the predetermined direction is the circumferential direction of the pant leg. In other words, the second electrode 2241 is a unidirectional deformation electrode or a unidirectional strain-sensitive electrode adapted to a circumferential deformation of the pant leg. When the user wears the pants 2100, the second electrode 2241 deforms only along the circumferential direction of the pant leg. For example, an amount of deformation (e.g., an extended length f3) of the second electrode 2241 along the circumferential direction of the pant leg is much larger than an amount of deformation (e.g., an extended length f4) of the second electrode 2241 in other directions (directions other than the circumferential direction). For example, f3 is more than 10 times of f4. Alternatively, when the user puts on the pants 2100, the second electrode 2241, while deforming in various directions, only responds to the deformation along the circumferential direction of the pant leg to generate a change in the resistance, i.e., the second electrode 2241 is sensitive to a force in only a single direction (i.e., the circumferential direction of the pant leg), and generates almost no change in resistance in response to deformations in directions other than the circumferential direction of the pant leg. For example, a change e3 in the resistance of the second electrode 2241 generated in response to an amount of deformation of the pant leg along the circumferential direction thereof is much larger than a change e4 in the resistance of the second electrode 2241 generated in response to the same amount of deformation of the pant leg in other directions (directions other than the circumferential direction). For example, e3 is more than 10 times of e4.

In some embodiments, as shown in FIG. 2B and FIG. 10, the sensor assembly 2200 further includes a third electrode module 2250, the third electrode module 2250 including at least one third electrode 2251. In some embodiments, the predetermined direction is a length direction of the pant leg. In other words, the third electrode 2251 is a unidirectional deformation electrode or a unidirectional strain-sensitive electrode adapted to a deformation of the pant leg along the length direction of the pant leg. When the user wears the pants 2100, the third electrode 2251 deforms only along the length direction of the pant leg. For example, an amount of deformation (e.g., an extended length f5) of the third electrode 2251 along the length direction of the pant leg is much larger than an amount of deformation (e.g., an extended length f6) of the third electrode 2251 in other directions (directions other than the length direction). For example, f5 is more than 10 times of f6. Alternatively, when the user wears the pants 2100, the third electrode 2251, while deforming in various directions, only generates a change in the resistance in response to the deformation of the pant leg in the length direction of the pant leg, i.e., the third electrode 2251 is only sensitive to a force in a single direction, and generates almost no change in resistance in response deformations in directions other than the length direction of the pant leg. For example, a change e5 in the resistance of the third electrode 2251 generated in response to the amount of deformation of the pant leg in the length direction is much larger than a change e6 in the resistance of the third electrode 2251 generated in response to the same amount of deformation of the pant leg in other directions (directions other than the length direction). For example, e5 is more than 10 times of e6. The third electrode module 2250 is configured such that the amount of deformation of the pant leg along its length direction can be accurately measured, thereby providing a reference for determining the shape of the leg of the user.

The third electrode 2251 may be configured as a unidirectional deformation electrode or a unidirectional strain-sensitive electrode adapted to the deformation of the pant leg along the length direction of the pant leg in a manner similar to the way in which the second electrode 2241 is configured as a unidirectional deformation electrode or a unidirectional strain-sensitive electrode adapted to the deformation of the pant leg along the circumferential direction of the pant leg.

In some embodiments, the sensor assembly 2200 may include a fourth electrode module. The fourth electrode module may include at least two fourth electrodes (not shown in the figures) configured as unidirectional deformation electrodes or unidirectional strain-sensitive electrodes for deformations of different directions of the pant leg. For example, one of the at least two fourth electrodes may be a unidirectional strain-sensitive electrode adapted to the deformation of the pant leg in the length direction of the pant leg, and another one of the at least two fourth electrodes may be a unidirectional strain-sensitive electrode adapted to the deformation of the pant leg along the circumferential direction of the pant leg. In such cases, deformations in two directions of the pant leg can be accurately measured through a single electrode module.

In some embodiments, to enhance the wearing comfort of the user and adapt to the needs of different scenarios, regions of the pant leg where different strain sensors are located may have different elasticities. In some embodiments, an elasticity coefficient of the calf region 2120 of the pant leg in which the second strain sensor 2212 is located may be relatively large to prevent varicose veins in the calf region. For example, the elasticity coefficient of the calf region 2120 of the pant leg in which the second strain sensor 2212 is located is greater than the elasticity coefficient of the thigh region 2110 of the pant leg in which the first strain sensor 2211 is located. As another example, to make the waist region 2130 more comfortable, an elasticity coefficient of the waist region 2130 of the pants in which the third strain sensor 2213 is located may be relatively small. For example, the elasticity coefficient of the waist region 2130 in which the third strain sensor 2213 is located is smaller than the coefficient of elasticity of the thigh region 2110 of the pant leg where the first strain sensor 2211 is located. In some embodiments, the elasticity coefficient of the corresponding region on the pants may be changed by changing the way the pants are cut and the material configuration of the pants (e.g., the tailoring and material configuration of the thigh region 2110 and the calf region 2120), and/or by modifying a structure or a material of the strain sensor. For example, under the condition of the same material of the pant leg, a strain sensor that is less prone to deformation (e.g., has a relatively large elasticity coefficient) may be provided in the calf region 2120 and a strain sensor that is more prone to deformation (e.g., has a relatively small elasticity coefficient) may be provided in the thigh region 2110, such that after the user wears the pants 2100, a force exerted by the pants 2100 on the calf region 2120 is greater than a force exerted by the pants 2100 on the thigh region 2110.

In some embodiments, referring to FIG. 2A and FIG. 2B, the wearable device 2000 may further include a processing circuit 2400. The processing circuit 2400 may be integrated in the pants 2100. In some embodiments, the processing circuit 2400 may be integrated in the waist region 2130 of the pants 2100. In some embodiments, the processing circuit 2400 may be the processing device 1100 in FIG. 1. In other embodiments, the processing circuit 2400 may be part of the processing device 1100 in FIG. 1. The processing circuit 2400 may be configured to process or send the circumferential dimension of the leg of the user. For example, the processing circuit 2400 may transmit the circumferential dimension of the leg of the user to the storage device 1300 or the one or more terminal devices 1400 via the network 1200. In some embodiments, the processing circuit 2400 may directly process the circumferential dimension to generate feedback information, which may be presented to the user. The feedback information may include circumference information of the leg of the user, circumference change information, a model of the leg of the user, training instruction recommendations, postural information, corrective recommendations, or the like.

The present disclosure also provides a wearable device that includes the pants 2100 and the sensor assembly 2200 as described in any of the aforementioned embodiments, and a processor 3000. The processor 3000 is configured to process data (e.g., circumferential dimensions, the shape of the leg, the EMG signal, etc.) measured by the sensor assembly (e.g., the circumferential strain sensor 2210, the longitudinal strain sensor 2220, the first electrode module 2230, the second electrode module 2240, or the third electrode module 2250). In some embodiments, the processor 3000 may control the wearable device 2000 to perform a predetermined operation. For example, the processor 3000 may control the sensor assembly 2200 to measure a circumferential dimension of the leg of the user. As another example, the processor 3000 may control the sensor assembly 2200 to measure the circumferential dimension of the leg of the user at regular intervals. In some embodiments, the processor 3000 may be the processing device 1100 described in FIG. 1. In some embodiments, processor 3000 may include the processing circuit 2400 as described above. The processor 3000 may be integrated into the pants 2100 (e.g., the waist region 2130 of the pants 2100) or may be located in a cloud to access the data collected by the sensor assembly 2200 through remote communication with the sensor assembly 2200.

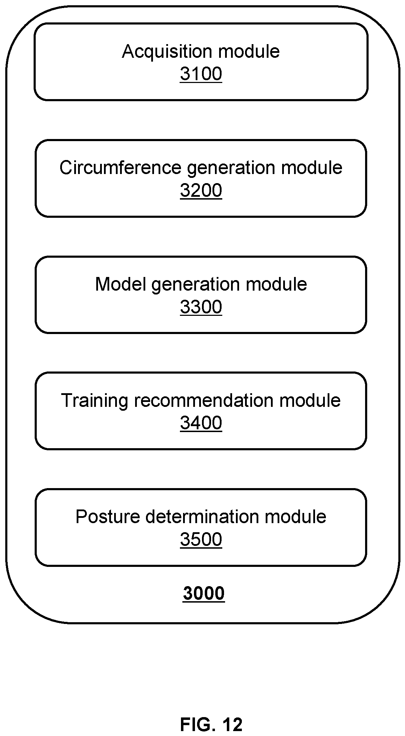

FIG. 12 is a block diagram of a processor of a wearable device according to some embodiments of the present disclosure. In some embodiments, as shown in FIG. 12, the processor 3000 may include an acquisition module 3100, a circumference generation module 3200, a model generation module 3300, a training recommendation module 3400, and a posture determination module 3500. It should be noted that the above modules are intended to be illustrative, and are not meant to limit the functionality of the processor 3000. For example, the processor 3000 may include only one or more of these modules, or the processor 3000 may include other modules.

In some embodiments, the processor 3000 may be configured to obtain a circumferential dimension of the leg of a user, and based on the circumferential dimension, generate circumference information of the leg of the user. The operation of obtaining the circumferential dimension of the leg of the user may be performed by the acquisition module 3100, and the operation of generating the circumference information of the leg of the user may be performed by the circumference generating module 3200. In some embodiments, the acquisition module 3100 may acquire the circumferential dimension of the leg of the user from the sensor assembly 2200 (e.g., the circumferential strain sensor 2210 or the second electrode module 2240). The circumference information may reflect the circumference of the leg of the user, and the circumference information may include circumferential dimensions of a plurality of parts (e.g., a thigh, a calf, a knee, an ankle, etc.) of the leg of the user. In some embodiments, the generated circumference information may be sent to the one or more terminal devices 1400. In some embodiments, the circumference information may be stored in the storage device 1300.

In some embodiments, the circumference information includes information about a change in the circumference over a first preset time period. The circumference change information may include data such as a trend (e.g., a trend of increase or decrease) and an amount of change in the circumferential dimension of the leg of the user over the first preset time period. The first preset time period may be a day, a week, a month, a year, or the like. Alternatively, the first preset time period may be a period of time defined by the user. The processor 3000 may be configured to monitor the circumferential dimension of the leg of the user during the first preset time period and generate the circumference change information. That is, the processor 3000 may control the sensor assembly 2200 to collect (e.g., once every interval of time) circumferential dimensions of the leg of the user multiple times during the first preset time period, and generate the circumference change information based on the plurality of circumferential dimensions collected multiple times. Merely by way of example, the processor 3000 may control the sensor assembly 2200 to collect circumferential dimensions of the thigh region 2110 of the leg of the user (e.g., via the first strain sensor 2211) every day for a month, and then based on the circumferential dimensions in the month, determine a trend of change, an amount of change, or the like in the circumference of the leg of the user. By generating the circumference change information and feeding it back to the user, the user can more easily understand the change in the circumference of his or her leg.

In some embodiments, the sensor assembly 2200 detects the shape of the leg of the user via the longitudinal strain sensor 2220. The processor 3000 may be configured to generate a model of the leg of the user based on the shape and the circumferential dimension of the leg of the user. This operation may be performed by the model generation module 3300. Exemplary models of the leg may include a two-dimensional (2D) image model, a three-dimensional (3D) image model, or the like. In some embodiments, the model generation module 3300 sends the generated model of the leg of the user to the one or more terminal devices 1400. In some embodiments, the generated model of the leg of the user may be stored in the storage device 1300. By generating the model of the leg of the user, the user can more conveniently and intuitively observe the shape of his or her leg, and thus perform targeted training to improve the shape of the leg.

In some embodiments, the processor 3000 may be configured to generate a first training guidance recommendation based on the circumference information of the leg of the users. This operation may be performed by the training recommendation module 3400. The first training guidance recommendation may be a training guidance recommendation for targeted training based on the circumference information of the leg of the user. In some embodiments, the first training guidance recommendation may be a training guidance recommendation that targets training for one or more specific parts of the leg. In some embodiments, the first training guidance recommendation may include a link to a training session, a training guidance video, etc. For example, if it is analyzed, based on the circumference information of the leg of the user, that the circumferential dimension of the thigh of the user is larger than a preset thigh circumferential dimension, the first training guidance recommendation for targeted training of the thigh may be fed back to the user. Furthermore, a link to a training course or a training instruction video for targeted training of the thigh may also be sent to the user. In this way, based on specific data of the user, the user may be scientifically guided to train in a targeted manner to achieve better shaping or fitness results.

In some embodiments, the processor 3000 may further adjust the first training guidance recommendation based on the circumference change information. For example, after the user has been training for a period of time, if the circumference change information shows that the circumference change of the user's training part (e.g., a thigh) is not optimal, the processor 3000 may modify the first training guidance recommendation (e.g., replacing the link to a training session with a more intense workout).

In some embodiments, the wearable device 2000 further includes the first electrode module 2230 integrated into the pants 2100, and the processor 3000 may be configured to generate, based on the circumferential dimension of the leg of the user and an EMG signal, a second training guidance recommendation. The second training guidance recommendation may be a training guidance recommendation generated based on the EMG signal of the user during exercise. The EMG signal may reflect the activity and function of the muscle during the user's exercise, and based on the analysis of the EMG signal, the user may be provided with training guidance recommendations from the perspective of training intensity. Exemplary second training guidance recommendations may include at least one of a fatigue reminder, a recommendation for a running exercise amount, a recommendation for target muscle strength training, or the like. Merely by way of example, the processor 3000 may determine, based on the circumferential dimension of the leg of the user, a part of the leg of the user that needs targeted training, and determine a corresponding training session. Further, the processor 3000 may adjust, based on the EMG signal of the user during exercise, the training intensity of the training session. The training session may be related to the training intensity of a target muscle or a target muscle group. The processor 3000 may extract one or more characteristic values from the EMG signal that characterize the intensity of muscle training, and determine whether the characteristic values satisfy a predetermined condition (e.g., the characteristic values are greater than a certain threshold) to reflect whether the user's current exercise satisfies a desired training effect for the target muscle or the target muscle group. If the predetermined condition is not satisfied, the processor 3000 may generate a recommendation to remind the user to adjust his or her movement or exertion technique. Exemplary characteristic values characterizing the intensity of muscle training may include amplitudes of the EMG signal, an absolute value of the amplitudes, an average value of the amplitudes (or the absolute value), a maximum value of the amplitudes, a minimum value of the amplitudes, a median frequency, a peak frequency, a total value of force exertion, an EMG power, a foot touchdown time, an average frequency, or the like, during a target time interval.

In some embodiments, during the user's training based on the first training guidance recommendation, the processor 3000 may generate the second training guidance recommendation by adjusting the first training guidance recommendation based on the EMG signal. For example, the processor 3000 may adjust a count of repetitions of an action in the first training guidance recommendation to generate the second training instruction recommendation. In other embodiments, the second training guidance recommendation may be generated based on the first training guidance recommendation and historical EMG signals of the user. For example, the processor 3000 may generate the second training guidance recommendation by adjusting the first training guidance recommendation based on the first training guidance recommendation in conjunction with the EMG signal of the user collected from a previous training session. In some embodiments, the generated first training guidance recommendation and the second training guidance recommendation may be sent to the one or more terminal devices 1400. In some embodiments, the generated first training guidance recommendation and the second training guidance recommendation may be stored in the storage device 1300.

In some embodiments, the processor 3000 may be configured to determine posture information reflecting a posture of the leg of the user based on the shape and/or the circumferential dimension of the leg of the user. This operation may be performed by the posture determination module 3500. The posture information includes a current posture of the leg of the user and a duration for which the leg of the user is in the current posture. Since the exertion of leg muscles varies in different postures, the shape and the circumferential dimension of the leg of the user may be different. By comparing and analyzing a current shape and a current circumferential dimension of the leg of the user with shapes and circumferential dimensions of the leg of the user in different postures, the posture information reflecting the current posture of the leg of the user can be determined. The posture information may be information related to a posture of the user, and the posture may include one or more of a standing posture, a sitting posture, a walking posture, an exercising posture, a lying down posture, and a preset improper posture (e.g., stilted legs, cross-legged sitting, kneeling, etc.).

In some embodiments, the duration for which the leg of the user is in the current posture is the total duration for which the leg of the user is in the current posture in a specific time period. For example, the processor may determine the total duration for which the user is in the sit-to-stand posture in a day. The specific time period may be a time period such as a day, a week, or a period of time defined by the user. Determining the duration for which the leg of the user is in the current posture and feeding it back to the user may enable the user to understand the duration for which the user is in the current posture in a specific time period, and thus to understand his or her activities during the time period. For example, the user may track how long he or she has been sitting throughout a day to assess whether he or she has been sitting for an excessively long time. In some embodiments, the processor may generate a reminder message in response to the leg of the user being in the current posture for a period longer than a predetermined duration, and feedback the reminder message to the user. For example, when the current posture of the user is a sit-to-stand posture, the processor generates a reminder message when the user is in the sit-to-stand posture for longer than a predetermined duration (e.g., one hour), and feeds the reminder message to the user.

In some embodiments, the posture includes at least a preset improper posture. The preset improper posture may include a crossed-leg posture, sitting cross-legged in a kneeling position, or the like. In some embodiments, the processor 3000 may be configured to: in response to determining that the current posture of the leg of the user is the preset improper posture, determine the duration for which the leg of the user is in the preset improper posture based on the shape of the leg of the user and/or the circumferential dimension of the leg of the user, and generate a corrective recommendation based on the duration for which the leg of the user is in the preset improper posture. In some embodiments, the above operation may be performed by the posture determination module 3500. When the user is in the preset improper posture, some specific parts of the leg of the user are continuously pressurized, resulting in a significant change in the shape of the leg and a change in the circumferential dimension of the leg. Thus, based on characteristics of the shape the change in the circumferential dimension of the specific parts of the leg in different preset improper postures, whether the user is in a preset improper posture and which preset improper posture the user is in can be determined. For example, when the user is in the kneeling posture, back sides of both the thigh and the calf may be compressed and become flattened, and an angle between the thigh and the calf may be relatively small. When the shape and the circumferential dimension of the leg of the user reflect this condition, it may be determined that the user is in the kneeling posture. The corrective recommendation includes a reminder that the user has maintained the preset improper posture for too long, a warning about the hazards of the improper posture, and a corrective suggestion for the improper posture (e.g., advice on correct sitting posture). For example, if the duration for which of the leg of the user is in the crossed-leg posture exceeds a preset time, the processor 3000 may generate a corrective recommendation. In some embodiments, the generated corrective recommendation may be sent to the one or more terminal devices 1400. In some embodiments, the generated corrective recommendation may be stored in the storage device 1300.

In some embodiments, the posture includes at least a walking posture and a running posture. In some embodiments, the processor 3000 may be configured to: in response to determining that the current posture of the leg of the user is the walking posture or the running posture, determine at least one of a step count, a step length, or a step frequency of the user based on the shape of the leg of the user and/or the circumferential dimension of the leg of the user. When the user is in the walking posture or the running posture, the shape and the circumferential dimension of the leg of the user may exhibit obvious periodic changes. Whether the user is in the walking posture or the running posture may be determined based on the changes. Merely by way of example, the step frequency of the user may be determined based on how often the shape and the circumferential dimension of the leg of the user show significant periodic changes, and the step length of the user may be determined based on a change in the shape of a specific part (e.g., a thigh and a calve) of the leg and a change in an angle between the thigh and the calf. By determining the step count, the step length, and/or the step frequency of the user, the user can more conveniently understand his or her walking or running situation.

In some embodiments, the processor may generate a reminder or a recommendation based on at least one of the step counts, the step length, or the step frequency of the user. For example, the processor may generate a recommendation for the user to perform more walking exercises in response to determining that the step count of the user is less than a preset step count threshold (e.g., 2,000 steps) over a period of time (e.g., a day).

In some embodiments, the posture includes at least a workout posture. The processor 3000 may be configured to: in response to determining that the current posture of the leg of the user is the workout posture, counting workout movements of the leg of the user based on the shape of the leg of the user and/or the circumferential dimension of the leg of the user. This operation may be performed by the posture determination module 3500. When the user is in the workout posture where the shape of the leg is constantly changing and the muscles constantly exert force, the circumferential dimension of the leg of the user changes constantly. Thus, based on measurements of the shape and the circumferential dimension of the leg of the user, whether the user is in the workout posture is determined. Counting the workout movements of the leg of the user may include determining the workout movements of the leg of the user and determining a count of repetitions of each workout movement. Based on the shape and the circumferential dimension of the leg of the user, the processor 3000 may determine the workout movements (e.g., jumping jacks, squats, etc.) being performed by the user, and when the movements are performed repeatedly mode, the processor 3000 may obtain the count of repetitions of each workout movement. Merely by way of example, the posture determination module 3500 may obtain the count of jumping jacks performed by the leg of the users. By counting the workout movements performed by the leg of the user, the user may have a clearer understanding of his or her workout. In some embodiments, a counting result of the workout movements of the leg of the user may be sent to the one or more terminal devices 1400. In some embodiments, the counting result of the workout movements of the leg of the user may be stored in the storage device 1300.

In some embodiments, the processor may generate a reminder or a recommendation based on the counting result of the workout movements of the leg of the user. For example, if the count of squats performed by the leg of the user is greater than a preset count, the processor may generate a reminder to alert the user to reduce the training intensity. In some embodiments, the processor may generate the reminder or the recommendation based on the counting result of workout movements of the leg of the user and an EMG signal. For example, after the user performs a training session, the processor determines the count of squats performed by the leg of the user during the training session. If the processor determines, based on an analysis of the EMG signal collected during the training session, that the user's training is excessively intense, the processor may generate a recommendation for adjusting the count of squats (e.g., recommending that the count of squats be adjusted from 40 to 20).

Having thus described the basic concepts, it may be rather apparent to those skilled in the art after reading this detailed disclosure that the foregoing detailed disclosure is intended to be presented as illustrative example and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of the present disclosure.

Moreover, certain terminology has been configured to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This way of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties configured to describe and claim certain embodiments of the present disclosure are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameter set forth in the written description and attached claims are approximations that may vary depending upon the desired properties sought to be obtained by a particular embodiment. In some embodiments, the numerical parameter should be construed in light of the number of reported significant digits and by applying ordinary rounding techniques. Notwithstanding that the numerical ranges and parameter setting forth the broad scope of some embodiments of the present disclosure are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the present disclosure disclosed herein are illustrating of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A wearable device, comprising:
pants; and
a sensor assembly fixed on the pants, wherein the sensor assembly includes at least one circumferential strain sensor, the at least one circumferential strain sensor extends along a circumferential direction of a pant leg of the pants and is configured to measure a circumferential dimension of a leg of a user.

2. The wearable device of claim 1, wherein the at least one circumferential strain sensor includes:
a first strain sensor, the first strain sensor being disposed in a thigh region of the pant leg and extending along a circumferential direction of the thigh region to measure a circumferential dimension of a thigh of the user; and
a second strain sensor, the second strain sensor being disposed in a calf region of the pant leg and extending along a circumferential direction of the calf region to measure a circumferential dimension of a calf of the user.

3. The wearable device of claim 2, wherein
the at least one circumferential strain sensor includes a plurality of first strain sensors, the plurality of first strain sensors being spaced apart in a length direction of the pant leg; and/or
the at least one circumferential strain sensor includes a plurality of second strain sensors, the plurality of the second strain sensors being spaced apart in the length direction of the pant leg.

4. The wearable device of claim 1, wherein the at least one circumferential strain sensor includes a third strain sensor, the third strain sensor is disposed in a waist region of the pants and extends along a circumferential direction of the waist region to measure a circumferential dimension of the waist of the user.

5. The wearable device of claim 2, wherein the sensor assembly further includes at least one longitudinal strain sensor, the longitudinal strain sensor extends along a length direction of the pant leg, at least a portion of the longitudinal strain sensor is located in the thigh region, and at least another portion of the longitudinal strain sensor is located in the calf region.

6. The wearable device of claim 5, wherein the at least one longitudinal strain sensor and the at least one circumferential strain sensor are located on a same bearing layer, and the at least one longitudinal strain sensor and the at least one circumferential strain sensor are arranged adjacent to each other.

7. The wearable device of claim 6, wherein a first gap is formed between two ends of the first strain sensor, a second gap is formed between two ends of the second strain sensor, and the longitudinal strain sensor passes through the first gap and the second gap.

8. The wearable device of claim 5, wherein the pants include at least two bearing layers stacked in a thickness direction of the pants, and the at least one circumferential strain sensor and the at least one longitudinal strain sensor are disposed on different bearing layers of the at least two bearing layers.

9. The wearable device of claim 5, wherein the at least one longitudinal strain sensor includes a plurality of longitudinal strain sensors, the plurality of longitudinal strain sensors are spaced apart along the circumferential direction of the pant leg.

10. The wearable device of claim 5, wherein the at least one longitudinal strain sensor is located in a region of the pant leg that faces towards or is away from the other pant leg of the pants.

11. The wearable device of claim 2, wherein an elasticity coefficient of the calf region of the pant leg in which the second strain sensor is located is greater than an elasticity coefficient of the thigh region of the pant leg in which the first strain sensor is located.

12. The wearable device of claim 1, wherein
the sensor assembly further includes a first electrode module integrated in the pants,
the first electrode module includes at least two first electrodes in contact with a skin of the user, the at least two first electrodes are spaced apart along a muscle fiber direction of a same muscle of the leg of the user; and
the first electrode module is configured to collect an electromyographic (EMG) signal of the muscle of the leg of the user.

13. The wearable device of claim 12, wherein the at least one circumferential strain sensor is arranged between the at least two first electrodes of the first electrode module.

14. The wearable device of claim 1, further comprising a processing circuit, wherein
the processing circuit is integrated in a waist region of the pants; and
the processing circuit is configured to process or send the circumferential dimension of the leg of the user.

15. A wearable device, comprising:
pants; and
a sensor assembly fixed on the pants, wherein the sensor assembly includes a second electrode module, the second electrode module includes at least one second electrode, the at least one second electrode extends along a circumferential direction of a pant leg of the pants, and the second electrode module is configured to measure a circumferential dimension of a leg of a user.

16. The wearable device of claim 15, wherein
the at least one second electrode includes at least two second electrodes in contact with a skin of the user, and the at least two second electrodes are spaced apart along a muscle fiber direction of a same muscle of the leg of the user; and
the second electrode module is configured to collect an EMG signal of the muscle of the leg of the user.

17. The wearable device of claim 16, wherein each of the at least two second electrodes is provided with a first signal lead-out end, a second signal lead-out end, and a third signal lead-out end,
the first signal lead-out end and the second signal lead-out end are configured to lead out a signal of the circumferential dimension of the leg of the user,
the first signal lead-out end and the second signal lead-out end are located at two ends of an extension direction of the second electrode, respectively, and
the third signal lead-out end is configured to lead out a potential signal.

18. The wearable device of claim 15, wherein the second electrode is configured as a unidirectional deformation electrode or a unidirectional strain-sensitive electrode adapted to a circumferential deformation of the pant leg.

19. The wearable device of claim 15, wherein the sensor assembly further includes a third electrode module, the third electrode module includes at least one third electrode, and the third electrode is configured as a unidirectional deformation electrode or unidirectional strain-sensitive electrode adapted to a length direction of the pant leg.

20. A wearable device, comprising:
pants;
a sensor assembly fixed on the pants, wherein the sensor assembly includes at least one circumferential strain sensor, the at least one circumferential strain sensor extends along a circumferential direction of a pant leg of the pants and is configured to measure a circumferential dimension of a leg of a user; and
a processor, configured to:
generate circumference information of the leg of the user by obtaining the circumferential dimension of the leg of the user.

21. The wearable device of claim 20, wherein the circumference information includes circumference change information in a first preset time period; and
to generate the circumference information of the leg of the user by obtaining the circumferential dimension of the leg of the user, the processor is configured to:
monitor the circumferential dimension of the leg of the user in the first preset time period to generate the circumference change information.

22. The wearable device of claim 20, wherein the sensor assembly further includes at least one longitudinal strain sensor, the at least one longitudinal strain sensor extends along a length direction of the pant leg and is configured to detect at least a shape of the leg of the user; and
the processor is configured to:
generate a model of the leg of the user based on the shape and the circumferential dimension of the leg of the user.

23. The wearable device of claim 20, wherein the processor is configured to:
generate a first training guidance recommendation based on the circumferential dimension of the leg of the user.

24. The wearable device of claim 21, further comprising a first electrode module integrated in the pants, wherein
the first electrode module includes at least two first electrodes, the at least two first electrodes being spaced apart along a muscle fiber direction of a same muscle of the leg of the user;
the first electrode module is configured to collect an EMG signal of the muscle of the leg of the user; and
the processor is configured to generate a second training guidance recommendation based on the circumference change information of the leg of the user and the EMG signal.

25. The wearable device of claim 22, wherein the processor is configured to:
determine posture information reflecting a posture of the leg of the user based on the shape of the leg of the user and/or the circumferential dimension of the leg of the user, the posture information including a current posture of the leg of the user and a duration for which the leg of the user is in the current posture.

26. The wearable device of claim 25, wherein the posture includes at least a preset improper posture, and to determine the posture information reflecting the posture of the leg of the user based on the shape of the leg of the user and/or the circumferential dimension of the leg of the user, the processor is configured to:
in response to determining that the current posture of the leg of the user is the preset improper posture, determine the duration for which the leg of the user is in the preset improper posture based on the shape of the leg of the user and/or the circumferential dimension of the leg of the user; and
generate a corrective recommendation based on the duration for which the leg of the user is in the preset improper posture.

27. The wearable device of claim 25, wherein the posture includes at least a walking posture and a running posture, and the processor is configured to:
in response to determining that the current posture of the leg of the user is the walking posture or the running posture, determine at least one of a step count, a step length, or a step frequency of the user based on the shape of the leg of the user and/or the circumferential dimension of the leg of the user.

28. The wearable device of claim 25, wherein the posture includes at least a workout posture, and the processor is configured to:
in response to determining that the current posture of the leg of the user is the workout posture, count workout movements of the leg of the user based on the shape of the leg of the user and/or the circumferential dimension of the leg of the user.
